# EUROPEAN PATENT APPLICATION

(11) **EP 4 063 861 A1**
(43) Date of publication of application: **28.09.2022**
(21) Application number: 20890637.0
(22) Date of filing: 17.11.2020
(51) Int. Cl.: G01N 33/53

(54) **METHOD FOR COLLECTING DATA FOR PREDICTING OCCURRENCE OF ANAPHYLAXIS**

(30) Priority: 18.11.2019 JP 2019208045
(71) Applicant: Applied Medical Enzyme Research Institute Corporation, Tokushima-shi, Tokushima 770-0873 (JP)
(72) Inventor: KIDO, Hiroshi, Tokushima-shi, Tokushima 770-0873 (JP); SUZUKI, Koichi, Tokushima-shi, Tokushima 770-0873 (JP); TADA, Hitomi, Tokushima-shi, Tokushima 770-0873 (JP); SHINAHARA, Wakako, Tokushima-shi, Tokushima 770-0873 (JP)
(74) Representative: Script IP Limited
(86) International application number: PCT/JP2020/042862
(87) International publication number: WO 2021/100719

(57) **Abstract**

Provided is an approach of predicting a risk of occurrence of anaphylaxis in an infant or a child having food allergy by measuring an antibody titer of an IgE antibody and avidity of the IgE antibody against an allergen in a sample collected from the infant or the child, and predicting the risk of occurrence of anaphylaxis in the infant or the child according to a criterion created on the basis of ROC analysis.

## Description

### Technical Field

The present invention relates to a method for collecting data for predicting the possibility of occurrence of anaphylaxis in an infant or a child having allergy by using, as a criterion, a cutoff value determined by ROC analysis based on the degree of affinity (avidity) of an IgE antibody for an allergen, etc. in a sample collected from the infant or the child.

### Background Art

Food allergy is defined as a "phenomenon in which symptoms unfavorable to organisms are induced via antigen-specific immunological mechanisms caused by food" (see, for example, non-patent document 1). It is reported that oral immunological tolerance (immune tolerance) typically works on food to be digested or absorbed for the maintenance of humans' life so as not to cause the body's protective response to undigested foreign matter. However, babies of 5 to 10% of the birth population reportedly develop some food allergy by 1 year after birth. Neither the mechanism of occurrence thereof nor a method for predicting occurrence thereof has yet been elucidated completely.

Anaphylaxis is defined as "serious (not mild) life-threatening hypersensitive reaction that induces allergic symptoms throughout the body including a plurality of organs by the invasion of an allergen" (see, for example, non-patent document 2). Thus, the presence or absence of a risk of anaphylaxis becomes a considerable concern to patients having allergy.

Various methods for measuring antibody titers of antigen-specific IgE antibodies, including ImmunoCAP (Thermo Fisher Scientific, Inc.), are currently used for the diagnosis of allergy. However, it has been reported for ImmunoCAP that, for example, in the case of creating a probability curve which indicates the possibility of occurrence of allergy in test subjects, the possibility of occurrence of anaphylaxis exhibits a lower value than the actual incidence in infants of classes 2 and 3 with relatively low IgE antibody titers (see, for example, non-patent documents 3 and 4).

A method for collecting data for predicting a risk of occurrence of allergy to an allergen in a baby before occurrence of food allergy by respectively quantitatively measuring IgG1 and IgE antibody titers against the allergen in a plasma sample of the baby up to 6 months after birth, and referring to a criterion created on the basis of the degree of immunoglobulin class switching from IgG1 to IgE statistically processed in advance (see, for example, patent document 1) has been proposed and however, has the difficulty in predicting the occurrence of anaphylaxis.

"Oral food challenge (OFC)", which has been reported as the most reliable diagnosis method as to the occurrence of anaphylaxis in babies or children having food allergy, is practiced under the attendance of a doctor. Since there is a possibility that a test subject develops anaphylaxis, there is a strong demand for the development of a method for predicting the possibility of occurrence of anaphylaxis without OFC. However, there is no previous report on an effective diagnosis method or an effective biomarker, etc. for predicting the possibility of occurrence of anaphylaxis.

### Prior Art Documents

### Patent Document

Patent Document 1: International Publication No. WO2017/195871

### Non-Patent Documents

Non-patent Document 1: Guidelines for the diagnosis and management of food allergy, 2011, Japanese Society of Food Allergy
Non-patent Document 2: Simons FE, Ardusso LR, Bilo MB, EI-Gamal YE, Ledford DK, Ring J, Sanchez-Borges M, Senna GE, Sheikh A, Thong BY, World Allergy Organ J. 2011; 4: 13-37 Non-patent Document 3: Komata T, Soderstrom L, borres MP, Tachimoto H, Ebisawa M., J Allergy Clin Immunol. 2007; 119: 1272-1274
Non-patent Document 4: Furuya K, Nagao M, Sato Y, Ito S, Fujisawa T., Allergy. 2016; 71: 1435-1443

### Summary of the Invention

### Object to be Solved by the Invention

An object of the present invention is to provide an approach of predicting a risk of occurrence of anaphylaxis in an infant or a child having allergy.

### Means to Solve the Object

The present inventors have thought that in a situation where the occurrence of anaphylaxis cannot be effectively predicted by merely using an antibody titer of an IgE antibody as a parameter, another parameter may be necessary for predicting the occurrence of anaphylaxis. Specifically, the present inventors have thought that the following needs to be taken into consideration: blood contains an IgE antibody as well as an IgG1 antibody, an IgG2 antibody, an IgG3 antibody, an IgG4 antibody, an IgM antibody, an IgA antibody, an IgD antibody, and the like which react with the same allergen and each of these antibodies is a polyclonal antibody that recognizes a plurality of epitopes in an antigen.

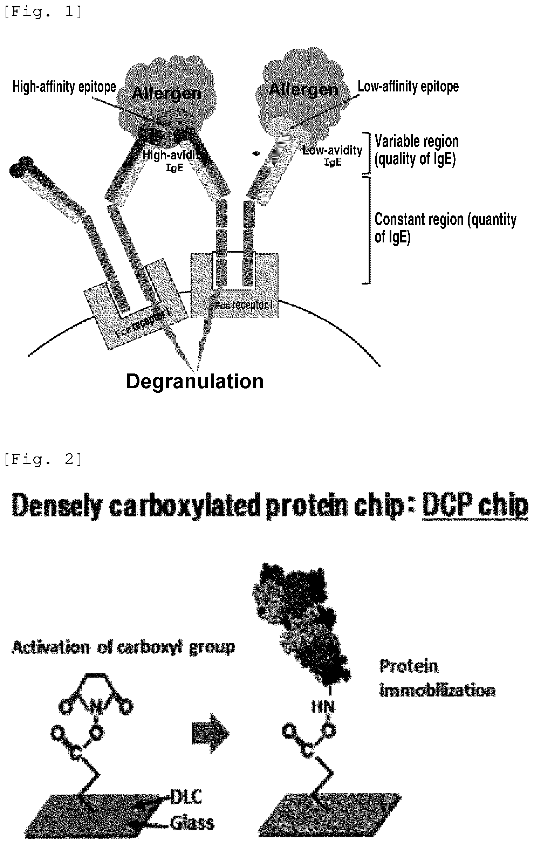

The present inventors have conceived the idea that in the case of evaluating the binding of the IgE antibody to an allergen, not only the measurement of an IgE antibody titer obtained by measuring only a previously measured constant region of the IgE antibody with a secondary antibody, i.e., the "quantity of IgE" at the constant region (Fc region) of the IgE antibody shown in Figure 1, but the "total affinity between an antigen and the antibody" (socalled avidity) as the "quality of IgE" which is exhibited by a variable region of the IgE antibody against an allergen needs to be taken into consideration. The present inventors selected a value of 1/IC50 as a parameter that indicates avidity of an IgE antibody against an allergen, analyzed the presence or absence of occurrence of anaphylaxis using a numerical value obtained by multiplying an IgE antibody titer of each allergen sensitization-positive infant or child by a 1/IC50 value, as the avidity value, and conducted a significant difference test of the parameter. As a result, the present inventors have found that this approach exhibits marked statistically significant difference between a subject with anaphylaxis and a subject without anaphylaxis, as compared with use of the existing parameter. In ROC (receiver operating characteristic) curve analysis which is used in the performance evaluation of examination, the numerical value obtained by multiplying an IgE antibody titer by a 1/IC50 value has also been found to have significant increase in area under curve (AUC) as a usefulness index value calculated from sensitivity and specificity thereof, as compared with conventional examination based on only the quantity of an IgE antibody. The present inventors have confirmed that the occurrence of anaphylaxis can be effectively predicted as to allergy patients for which there has been no effective diagnosis method for the possibility of occurrence of anaphylaxis so far, leading to the completion of the present invention.

Specifically, the present invention is defined by the following items.
[1] A method for collecting data for predicting a risk of occurrence of anaphylaxis in an infant or a child having food allergy by using, as a criterion, a cutoff value determined by ROC analysis based on avidity of an IgE antibody against an allergen in a sample collected from the infant or the child.
[2] The method for collecting data according to [1], wherein the cutoff value determined by ROC analysis based on avidity of an IgE antibody against an allergen is a cutoff value determined by ROC analysis based on a numerical value obtained by multiplying the avidity of an IgE antibody against an allergen by an antibody titer of the IgE antibody against the allergen.
[3] The method for collecting data according to [2], wherein the avidity of an IgE antibody against an allergen is numerically converted as 1/IC50.
[4] The method for collecting data according to [3], wherein the IC50 is a competitive binding inhibitory activity value.
[5] The method for collecting data according to [1], wherein the cutoff value determined by ROC analysis based on avidity of an IgE antibody against an allergen is a cutoff value determined by ROC analysis based on IC50 of the IgE antibody.
[6] The method for collecting data according to [5], wherein the IC50 is a competitive binding inhibitory activity value.
[7] The method for collecting data according to any one of [2] to [6], wherein the antibody titer of the IgE antibody against the allergen is measured using a DCP chip.
[8] The method for collecting data according to any one of [1] to [7], wherein the sample is plasma or serum.

### Effect of the Invention

According to the present invention, the possibility of occurrence of anaphylaxis can be significantly predicted as to an infant or a child for which the occurrence of anaphylaxis has heretofore been difficult to diagnose.

### Brief Description of Drawings

[Figure 1] Figure 1 shows structural features that dictate the "quantity" and "quality" of an IgE antibody.
[Figure 2] Figure 2 shows a schematic view of a proteinimmobilized DCP chip.

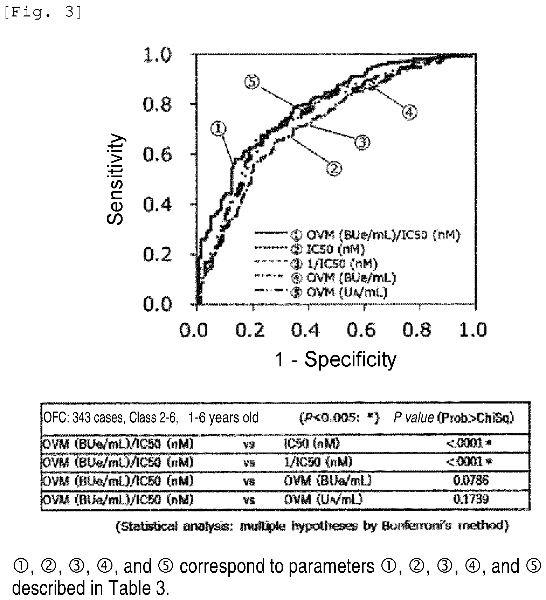

[Figure 3] Figure 3 shows, in a ROC curve, results of ROC analysis of 343 infants aged 1-6 years who were determined as class 2 or higher by the ImmunoCAP method, using five different measurement parameters: CAP OVM sIgE (UA/mL), DCP OVM sIgE (BUe/mL), DCP OVM sIgE/DCP OVM sIgE IC50 [(BUe/mL)/(nM)], DCP OVM sIgE IC50 (nM), and 1/DCP OVM sIgE IC50 (nM) for a heated chicken egg allergen ovomucoid (OVM), for allergy-positive subjects and -negative subjects in heated chicken egg OFC conducted.

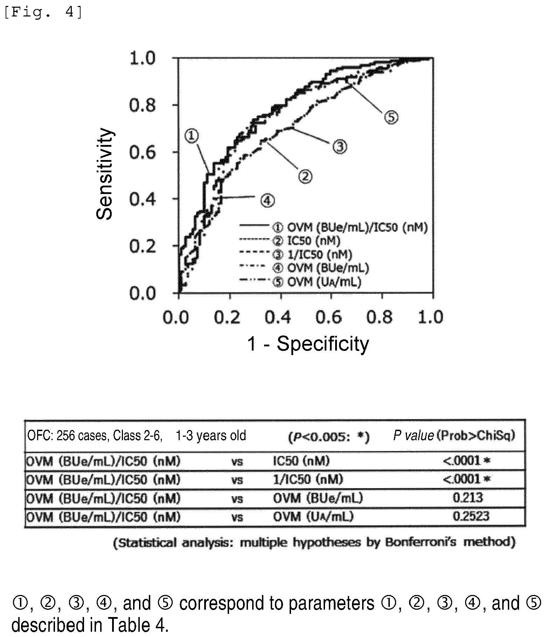

[Figure 4] Figure 4 shows, in a ROC curve, results of ROC analysis of 256 infants aged 1-3 years who were determined as class 2 or higher by the ImmunoCAP method, using five different parameters: CAP OVM sIgE (UA/mL), DCP OVM sIgE (BUe/mL), DCP OVM sIgE/DCP OVM sIgE IC50 [(BUe/mL)/(nM)], DCP OVM sIgE IC50 (nM), and 1/DCP OVM sIgE IC50 (nM), for allergy-positive subjects and -negative subjects in heated chicken egg OFC conducted.

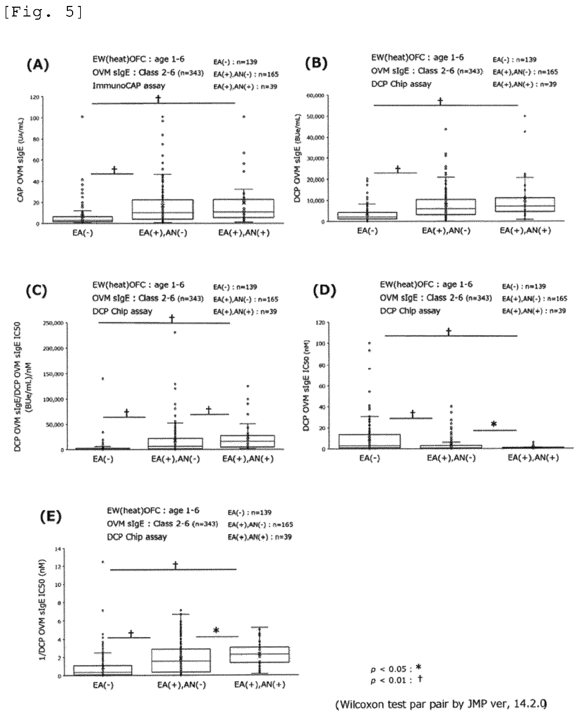

[Figure 5] Figure 5 shows results of testing the presence (+) or absence (-) of chicken egg allergy (EA) in 343 infants aged 1-6 years who were determined as class 2 or higher by the ImmunoCAP method, using five (A to E) different parameters: (A) CAP OVM sIgE (UA/mL), (B) DCP OVM sIgE (BUe/mL), (C) DCP OVM sIgE/DCP OVM sIgE IC50 [(BUe/mL)/ (nM)], (D) DCP OVM sIgE IC50 (nM), and (E) 1/DCP OVM sIgE IC50 (nM), for heated chicken egg [EW (heat)] OFC positive or negative, and also results of further testing significant difference between the presence (+) and absence (-) of anaphylaxis (AN) as to the subjects with chicken egg allergy (OFC-positive subjects).

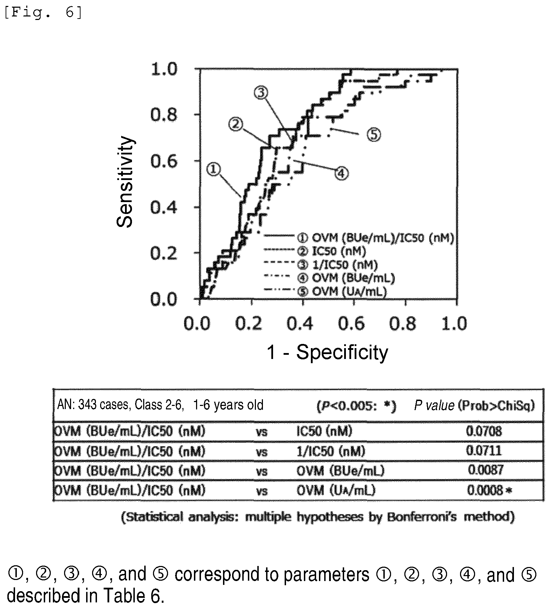

[Figure 6] Figure 6 shows results of ROC analysis of 343 infants aged 1-6 years who were determined as class 2 or higher by the ImmunoCAP method, using five different parameters: CAP OVM sIgE (UA/mL), DCP OVM sIgE (BUe/mL), DCP OVM sIgE/DCP OVM sIgE IC50 [(BUe/mL)/(nM)], DCP OVM sIgE IC50 (nM), and 1/DCP OVM sIgE IC50 (nM), and by relying on the diagnosis of anaphylaxis onset by OFC, for subjects diagnosed as anaphylaxis (AN+) cases and non-anaphylaxis (AN-)cases.

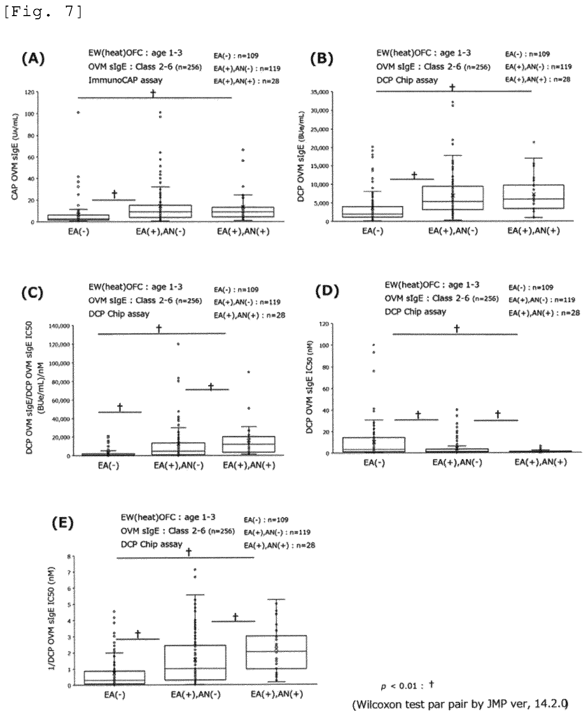

[Figure 7] Figure 7 shows results of testing the presence (+) or absence (-) of chicken egg allergy (EA) in 256 infants aged 1-3 years who were determined as class 2 or higher by the ImmunoCAP method, using five (A to E) different parameters: (A) CAP OVM sIgE (UA/mL), (B) DCP OVM sIgE (BUe/mL), (C) DCP OVM sIgE/DCP OVM sIgE IC50 [(BUe/mL)/(nM)], (D) DCP OVM sIgE IC50 (nM), and (E) 1/DCP OVM sIgE IC50 (nM) for OFC positive or negative, and results of further testing significant difference between the presence (+) and absence (-) of anaphylaxis (AN) in OFC as to the subjects with chicken egg allergy (OFC-positive subjects).

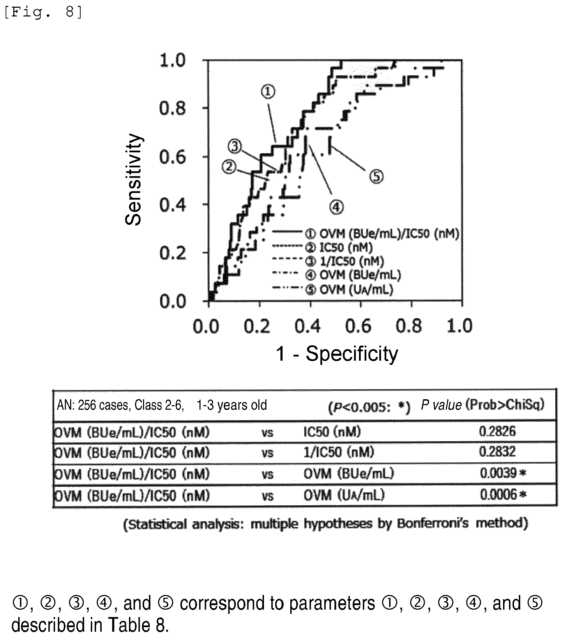

[Figure 8] Figure 8 shows results of ROC analysis of 256 infants aged 1-3 years who were determined as class 2 or higher by the ImmunoCAP method, using five different parameters: CAP OVM sIgE (UA/mL), DCP OVM sIgE (BUe/mL), DCP OVM sIgE/DCP OVM sIgE IC50 [(BUe/mL)/(nM)], DCP OVM sIgE IC50 (nM), and 1/DCP OVM sIgE IC50 (nM), and by relying on the diagnosis of anaphylaxis onset by OFC, for subjects diagnosed as anaphylaxis (AN+) cases and non-anaphylaxis (AN-)cases.

### Mode of Carrying Out the Invention

The method for collecting data for predicting a risk of occurrence of anaphylaxis in an infant or a child having food allergy according to the present invention is not particularly limited as long as the method uses, as a criterion, a cutoff value determined by ROC analysis based on avidity of an IgE antibody against an allergen in a sample collected from the infant or the child having food allergy. Examples of the cutoff value determined by ROC analysis based on avidity of an IgE antibody against an allergen can include a cutoff value determined by ROC analysis by using, as a parameter, a numerical value obtained by multiplying the avidity of an IgE antibody against an allergen by an antibody titer of the IgE antibody against the allergen, and a cutoff value determined by ROC analysis by using IC50 of the IgE antibody as a parameter. These two cutoff values may be used in combination as a criterion. The method of the present invention may be nonmedically performed, i.e., also includes a method for nonmedically collecting data, and also includes a method for collecting data for predicting a risk of occurrence of anaphylaxis in an infant or a child having food allergy, which excludes the practice of a doctor. Examples of the food allergy can include allergic reaction caused by a specific food product capable of serving as an allergen. Examples of the anaphylaxis can particularly include serious life-threatening hypersensitive reaction among allergic reactions.

The allergen means an antigen that establishes allergen sensitization or triggers allergic reaction and further anaphylaxis. Specifically, the allergen is not particularly limited as long as the allergen is an arbitrary antigenic protein or peptide as well as a sugar chain, a nucleic acid, or the like capable of inducing the production of an IgE antibody in a human. Examples thereof can include an ingredient serving as a food allergen such as eggs, milks, meats such as beef, fishes such as salmon and tuna, crustaceans and mollusks such as shrimps and crabs, cereals, beans and nuts, fruits, vegetables, beer yeast, and gelatin. Among others, examples of the major allergen can include a milk allergen such as αs1-casein, αs2-casein, β-casein, κ-casein, α-lactalbumin, and β-lactoglobulin (BLG) which is a major component of a whey allergen, an egg white allergen (EW) which is ovomucoid (OVM), ovalbumin (OVA), conalbumin, or a mixture thereof, an egg yolk allergen, and an egg allergen as a mixture thereof, a wheat allergen such as gliadin and gluten, a buckwheat allergen, a peanut allergen such as Ara h1, a sesame allergen such as 11S globulin, and a crustacean allergen such as tropomyosin protein.

The allergen sensitization refers to a state where an allergen that has entered the body is recognized as foreign matter to be eliminated by immune function, which produces an IgE antibody that recognizes and binds to the allergen. Such a state is referred to as being sensitized to the allergen or as established allergen sensitization, etc. Mere allergen sensitization does not always cause allergic reaction.

Examples of the allergic reaction can include reaction in which when an allergen reinvades the body in a situation having the established allergen sensitization, an IgE antibody on a mast cell binds to the allergen so that a chemical mediator such as histamine is released from the mast cell. Symptoms caused by such allergic reaction are referred to as allergic symptoms.

Examples of the allergic symptom can include: a cutaneous symptom such as itching sensation, hives, angioedema, red flare, and eczema; an eye symptom such as conjunctival injection or edema, itching sensation, lacrimation, and lid edema; a nasal symptom such as sneezing, nasal discharge, and nasal congestion; an oropharyngeal symptom such as unpleasant feeling or swelling in the oral cavity, the lip, or the tongue, and itchy throat or irritating feeling in the throat; a digestive symptom such as abdominal pain, nausea, vomiting, diarrhea, and hematochezia; a respiratory symptom such as throat tightness, pharyngeal edema, hoarseness, coughing, stridor, dyspnea, and asthma; and a systemic symptom such as anaphylactic shock with tachycardia, collapsed state, disturbance of consciousness, and blood pressure reduction.

The sample according to the present invention is not particularly limited as long as the sample is collected from an infant or a child having allergy. Examples thereof can include a body fluid such as blood, serum, plasma, saliva, lacrimal fluid, nasal discharge, and urine collected from a patient. Serum or plasma is preferred. In the case of using serum as the sample, for example, blood is collected from the brachial vein of a patient, and the obtained blood is left standing overnight at 4°C and then centrifuged. The supernatant can be used as the serum. Alternatively, 50 to 100 µL of micro-volume blood obtained by the low-invasive needling of the earlobe or the fingertip with a micro blood collection needle or the like may be collected into a microcapillary tube and used as it is.

The infant or the child having food allergy is an infant or a child aged 1 year and 0 months (1-year-old) to 12 years, preferably a 1- to 6-year-old infant or child, more preferably a 1- to 3-year-old infant. Examples thereof can include an infant or a child who evidently manifests allergic symptoms by the ingestion of a food allergen, and an infant or a child determined as being allergy-positive and having food allergy by a method known in the art. Such an infant or a child includes an infant or a child who has been evidently sensitized to an allergen and has the possibility of occurrence of allergy and however, has not yet manifested allergic symptoms, and may include an infant or a child definitively diagnosed as being not an allergy-positive subject by OFC, a conventional definitive diagnosis method for food allergy.

Specific examples of the method for determining the presence of food allergy in the infant or the child can include a method of definitively diagnosing the presence or absence of allergy on the basis of a reference in each method in a skin prick test, a basophil activation test, IgE assay known in the art, or OFC.

### [Measurement of IgE antibody titer]

The method for quantitatively measuring the antibody titer of the IgE antibody is not particularly limited as long as the method, including the IgE assay known in the art, can detect the IgE antibody against an allergen in a sample and quantitatively measure the IgE antibody titer. Preferred examples thereof can include ELISA using a labeled secondary antibody which is performed on a carrier. Examples of the carrier in ELISA can include a commercially available chip for ELISA, a chip with a carbon layer formed on the surface of a carrier because of being advantageous in that nonspecific adsorption is low, and a chip containing a chemical modifying group and an activated chip because of being advantageous for peptide immobilization. Among others, a DCP chip can be preferred. Examples of the IgE assay known in the art can include a DCP method using the DCP chip, ImmunoCAP, Allastat 3g, IMM-FAST CHECK, and Oriton IgE. For calculating the antibody titer of IgE, it is also preferred that a blood sample such as serum should be diluted 1-fold to tens of folds, preferably 1.1- to 10-fold, more preferably 1.5- to 5-fold, and then subjected to the quantification of the IgE antibody titer, from the viewpoint of more accurate measurement within the range of accuracy of a calibration curve. In this case, the IgE antibody titer is measured by the DCP method using the DCP chip, etc. and then needs to be multiplied by the dilution ratio of the blood sample to finally determine the IgE antibody titer.

### [DCP method]

In the DCP method, examples of the method for determining the presence or absence of allergy in the targeted infant or child can include a method that can determine the presence of allergy on the basis of the degree of production of an IgE antibody, i.e., an IgE antibody titer, in a blood sample of the targeted infant or child and specifically determines the presence of allergy when the antibody titer against, for example, ovomucoid (OVM), is 800 BUe/mL or more, preferably 1000 BUe/mL or more, though the value differs depending on an allergen.

Examples of the DCP method can include a method of measuring an IgE antibody by ELISA using a DCP chip. Examples of the DCP chip can include a chip obtained by providing an electrostatic layer treated with an amino group-containing compound or a polymer and/or a copolymer thereof on the surface of a silicon substrate having a carbon layer treated with DLC (diamond-like carbon) or on the surface of a glass slide, and further treating the resultant with dicarboxylic acid or polyvalent carboxylic acid, etc., followed by activation with N-hydroxysuccinimide and/or carbodiimides, a chip containing a chemical modifying group introduced in the surface of a carrier or in the carbon layer, and a chip further activated.

Examples of the labeled secondary antibody can include a secondary antibody fluorescently labeled with HiLyte Fluor 555, Atto532, Cy3, Alexa Fluor 555, Cy5, FITC, or rhodamine, a secondary antibody enzymatically labeled with peroxidase or alkaline phosphatase, a magnetic bead-labeled secondary antibody, an infrared labeled secondary antibody, and a labeled anti-human IgE antibody. For example, a Fab fragment or a F(ab')₂ fragment of an antibody can also be used as the secondary antibody. The Fab fragment can be prepared by treating the antibody with papain or the like, and the F(ab')₂ fragment can be prepared by treating the antibody with pepsin or the like.

### [ImmunoCAP]

In the ImmunoCAP, examples of the method for determining the presence or absence of allergy in the targeted infant or child can include a method of classifying an IgE antibody titer of less than 0.35 UA/mL into class 0, 0.35 to 0.69 UA/mL into class 1, 0.70 to 3.49 UA/mL into class 2, 3.50 to 17.49 UA/mL into class 3, 17.50 to 49.99 UA/mL into class 4, 50.00 to 99.99 UA/mL into class 5, and 100 UA/mL or more into class 6, and determining a subject of class 0 as being negative and being not sensitized to an allergen; a subject of class 1 as being false positive and being sensitized to an allergen at a level that manifests no allergic symptom; and a subject of class 2 or higher as being positive. The unit "UA/mL" of the IgE antibody titer is the unit of the IgE antibody titer produced against an allergen, uniquely set by Phadia AB which developed ImmunoCAP, and is quantitatively the same as IU/mL.

### [OFC]

The OFC is a test to confirm the presence or absence of symptoms by the single ingestion or ingestion in a plurality of divided portions of a food product responsible for allergy or suspected of causing allergy according to a definition described in the "2016 Digest Version of Guidelines for Diagnosis and Management of Pediatric Food Allergy in Japan, Chapter 7" (https://www.jspaci.jp/allergy_2016/chap07.html) of Food Allergy Committee, Japanese Society of Pediatric Allergy and Clinical Immunology. The total amount of the food product to be ingested in a single portion or divided portions is referred to as a total load. As examples of an ingestion interval and a dividing method, the ingestion interval is 20 to 60 minutes, and gradually increased ingestion of one to five portions in 1 to 2 hours is proposed. Examples thereof can include: in the case of two portions, 1/4 (first) and 3/4 (second) of the total intake or 1/3 and 2/3 of the total intake at a 60-minute interval; in the case of three portions, 1/8, 3/8, and 1/2 at 30- to 60-minute intervals; in the case of five portions, 1/16, 1/16, 1/8, 1/4, and 1/2 at 20- to 40-minute intervals; and in the case of 6 portions, 2/100, 4/100, 8/100, 16/100, 32/100, and 38/100. Despite the assumption that this test targets infants and is practiced under the attendance of a doctor, the test is likely to cause anaphylaxis. Therefore, it is preferred to be careful and to gradually increase a load by dividing the total load to more portions, i.e., 5 or 6 or more portions.

Examples of the total load can include: 1/2 of a chicken egg for 1-year-old infants and one chicken egg for 2-year-old or older infants; 50 mL of milk for 1-year-old infants and 200 mL of milk for 2-year-old or older infants; and in the case of wheat, 50 g of udon noodle for 1-year-old infants and 200 g of udon noodle for 2-year-old or older infants.

In the OFC, a criterion for the determination of positivity for allergy is as follows.
(A) Within several minutes or several hours from the start of allergen loading,
   one or more of
   cutaneous symptoms - itch, hives, erythema, and angioedema; and
   mucosal symptoms - conjunctival injection, nasal discharge, sneezing, and unpleasant feeling in the throat.
(B) Within several minutes or several hours from the start of allergen loading,
   one or more of
   respiratory symptoms - coughing and stridor;
   digestive symptoms - abdominal pain, vomiting, and diarrhea; and
   systemic symptoms - facial pallor, blood pressure reduction, and decreased level of consciousness.
(C) Within several minutes or several hours from the start of allergen loading,
   30% or more reduction in systolic blood pressure based on the age of a child

A subject that falls into one or more of (A), (B), and (C) is determined as being positive to allergy to the specific allergen.

A criterion for the determination of developed anaphylaxis is as follows.
"1 Within several minutes or several hours from allergen loading, cutaneous or mucosal symptoms (e.g., systemic hives, itchiness, flush, and swelling of the lip, the tongue, or the uvula) appear, and
one or more of the following A) and B) are satisfied:
   A) respiratory disturbance (e.g., dyspnea, expiratory stridor, inspiratory stridor, reduction in PEF (peak expiratory flow), and hypoxia), and
   B) blood pressure reduction or related organ failure (e.g., hypotension, collapse, faint, and incontinence).
2 Within several minutes or several hours from allergen loading, two or more of the following symptoms C) to F) appear:
   C) cutaneous or mucosal symptoms (e.g., systemic hives, itchiness, flush, and swelling of the lip, the tongue, or the uvula),
   D) respiratory disturbance (e.g., dyspnea, expiratory stridor, inspiratory stridor, reduction in PEF (peak expiratory flow), and hypoxia),
   E) blood pressure reduction or related organ failure (e.g., hypotension, collapse, faint, and incontinence), and
   F) persistent gastrointestinal symptoms (e.g., colic and vomiting). "

The criterion for predicting a risk of occurrence of anaphylaxis in an infant or a child having food allergy according to the present invention is not particularly limited as long as the criterion is created using an antibody titer of an IgE antibody against an allergen and avidity of the IgE antibody against the allergen. It is preferred to use a value obtained by multiplying the avidity (1/IC50) of the IgE antibody by the antibody titer of the IgE antibody.

### [Calculation of avidity of IgE antibody against allergen]

Examples of the value of the avidity of an IgE antibody against an allergen according to the present invention can include a value numerically converted as the value of IC50 or 1/IC50 of the IgE antibody. The value of IC50 is a value also called competitive binding inhibitory activity value and can be obtained, for example, by calculating a value that indicates the concentration of an allergen at which antigen-antibody binding reaction is inhibited by 50% as competitive binding inhibitory activity between an immobilized allergen and a soluble allergen. This calculation method is preferred because the value of IC50 can be evaluated even in the presence of an IgG, IgA, IgD, and/or IgM antibody that recognizes the same antigen as that of IgE.

The method for calculating the IC50 value of the IgE antibody is not particularly limited as long as the method can conduct competitive binding assay of the IgE antibody. Examples thereof can include a measurement method by biochemical assay such as ELISA. Examples thereof for a monoclonal antibody or a purified IgE antibody can include Biacore which can measure the degree of interaction by use of surface plasmon resonance technology, and an affinity measurement method by isothermal titration calorimetry.

Examples of the method for calculating the IC50 value by the ELISA can include a method of measuring the affinity of the IgE antibody for an allergen by using a protein denaturant as a chaotropic agent which reduces the intermolecular attraction between an antigen and an antibody, and a measurement method by competitive ELISA. The chaotropic agent does not have high reproducibility because the chaotropic agent might induce reduction in intermolecular attraction not only between an antigen and an antibody but between an antigen and another antigen and an antigen-antibody binding inhibitory effect varies depending on the dilution ratio of serum for use as a specimen even if the concentration of the chaotropic agent is the same. On the other hand, the measurement of the IC50 value by competitive ELISA using a competitive binding inhibition reaction method which is performed by the addition of an allergen at varying concentrations is preferred because this approach is highly reproducible because the IC50 value does not vary depending on the dilution ratio of a sample, permits mutual comparison of IC50 values among different allergens, and can quantitatively compare the total binding activity of antibodies against allergens. Hence, it has been confirmed that for calculating the IC50 value, multiplication by the dilution ratio of a sample is not necessary even if a blood sample such as plasma or serum is diluted. Exemplary specific procedures of the ELISA using a competitive binding inhibition reaction method will be given below.

### (Preliminary reaction)

An allergen with known concentrations prepared in stages from a concentration of 0 (a sample has no antigen as a competitive binding inhibitor) to a concentration sufficient for the binding of the allergen to all IgE antibodies within a given time is added as a competitive binding inhibitor to a sample containing an IgE antibody that binds to an antigen-specific allergen, and reacted for a given time.

Examples of the given time for the preliminary reaction can include 15 minutes to 2 hours, preferably 30 minutes to 1 hour. Concentrations appropriately determined by those skilled in the art on the basis of the amount of the antibody present in the sample, etc. can be used as the known concentrations prepared in stages. Examples of the staged allergen concentrations (final concentrations) that are used in competitive inhibition and added into serum or plasma can include, but are not limited to, a combination of 0 nM, 0.1 nM, 1.0 nM, 10 nM, 100 nM, and 1000 nM, and a combination of 0 nM, 0.1 nM, 1.0 nM, 10 nM, 100 nM, and 200 nM. The ELISA using the competitive binding inhibition reaction method is preferably performed as to an allergen having a definitive molecular weight. As for an allergen having an unknown molecular weight whose allergen concentration cannot be adjusted by a molar concentration, the staged allergen concentrations can be adjusted by a unit such as mg/mL or µg/mL.

### (Competitive binding inhibition reaction)

A solution after the preliminary reaction is reacted by a method of, for example, applying the solution to an allergen-immobilized carrier so that a free IgE antibody (primary antibody) in the solution after the preliminary reaction binds to the immobilized allergen. Then, the removal of the primary antibody and a washing process are performed, and the resulting solution is then further reacted with a labeled secondary antibody. The amount of the IgE antibody bound is calculated.

The concentration of an allergen at which antigen-antibody binding reaction is inhibited by 50%, i.e., "IC50", is expressed as an antigen concentration at which the amount of the label on the labeled secondary antibody to be detected becomes 50% (half maximal inhibitory concentration) when the amount of the label is defined as 100% as to a solution having no competitor allergen (concentration: 0) in the preliminary reaction.

The avidity of the IgE antibody against an allergen can be expressed, as mentioned above, as "1/IC50", the reciprocal of the IC50 value, on the basis of the IC50 value. In the ELISA using the competitive binding inhibition reaction method, lower affinity of the IgE antibody in the sample results in a larger amount of a free primary IgE antibody present in the solution after the preliminary reaction. Therefore, a larger amount of the antibody binds to the immobilized allergen, leading to a larger IC50 value. On the other hand, higher affinity of the IgE antibody in the sample results in a smaller amount of a free primary IgE antibody present in the solution after the preliminary reaction. Therefore, a smaller amount of the IgE antibody binds to the immobilized allergen, leading to a smaller IC50 value. Thus, for the sake of convenience, the reciprocal of the IC50 value may be used because the avidity is regarded as a lower numerical value in the case of lower affinity of the IgE antibody and regarded as a higher numerical value in the case of higher affinity of the IgE antibody. Particularly, in the case of multiplying the antigen affinity (avidity) of IgE by an IgE antibody titer for evaluation, the reciprocal of the IC50 value is used.

Examples of the labeled secondary antibody for avidity calculation can include a secondary antibody fluorescently labeled with HiLyte Fluor 555, Atto532, Cy3, Alexa Fluor 555, Cy5, FITC, or rhodamine, a secondary antibody enzymatically labeled with peroxidase or alkaline phosphatase, a magnetic bead-labeled secondary antibody, and an infrared labeled secondary antibody. For example, a Fab fragment or a F(ab')₂ fragment of an antibody can also be used as the secondary antibody. The Fab fragment can be prepared by treating the antibody with papain or the like, and the F(ab')₂ fragment can be prepared by treating the antibody with pepsin or the like.

Examples of the carrier with high binding sensitivity on which the allergen is immobilized can include a commercially available chip for ELISA, a chip with a carbon layer formed on the surface of a carrier because of being advantageous in that nonspecific adsorption is low, and a chip containing a chemical modifying group and an activated chip because of being advantageous for peptide immobilization. Among others, a DCP chip is preferred.

A chip and a method known in the art can also be used as the allergen-immobilized chip and the method for quantitatively measuring the IgE antibody using this chip. For example, a method using a chip described in Japanese unexamined Patent Application Publication Nos. 2006-267058, 2006-267063, and 2015-169616, i.e., the DCP chip, can be included in the scope of the present invention.

### [ROC analysis]

The criterion created on the basis of an antibody titer of an IgE antibody and avidity of the IgE antibody against an allergen according to the present invention is not particularly limited as long as the criterion permits prediction of a risk of occurrence of anaphylaxis in an infant or a child. Preferred examples thereof can include prediction using a cutoff value by ROC analysis by using a "numerical value obtained by multiplying the avidity of an IgE antibody against an allergen by an antibody titer of the IgE antibody" as a parameter.

The ROC is an abbreviation of receiver operating characteristic or receiver-operator characteristic, etc. and is a method that is utilized for comparing the ability of an independent variable to predict an outcome which is a dichotomous variable or for setting the cutoff of an independent variable for an outcome.

In the present invention, examples of the method for comparing the ability of an independent variable to predict an outcome which is a dichotomous variable can include a method in which, considering that a true positive rate and a false positive rate for anaphylaxis-positive subjects vary according to varying set thresholds for anaphylaxis positivity as to each of different n types of parameters, n types of ROC curves are created by plotting the true positive rate on the ordinate and the false positive rate on the abscissa, and a parameter having a larger area under curve (AUC) is evaluated as being better and having higher ability to predict outcome.

Examples of the method for creating the ROC curve can include, in the case of selecting a certain parameter, a method in which, when an arbitrary threshold is set as to the value of anaphylaxis positivity in the classification of test subjects, (true positive rate and false positive rate), i.e., (sensitivity and 1 - specificity), can be plotted for the threshold according to the true positive rate (sensitivity) of positive subjects correctly classified into a positive group = TY / (TY + FN) and the false positive rate (1 - specificity) of negative subjects classified into a positive group = FY / (FY + TN) wherein TY represents that an anaphylaxis-positive test subject is correctly classified into Y, FY represents that an anaphylaxis-positive test subject is mistakenly classified into Y, TN represents that an anaphylaxis-negative test subject is correctly classified into N, and FN represents that an anaphylaxis-negative test subject is mistakenly classified into N, and further, ROC curves are created by conducting many plots on variously set thresholds.

The method for setting the cutoff value in the ROC analysis is not particularly limited as long as the method can enhance the ability to predict an outcome. Examples thereof can include a method of determining the maximum value of sensitivity - (1 - specificity) as a cutoff value. The cutoff value thus set can also be determined by procedures known in the art using the following ROC analysis apparatus or commercially available software.

The ROC analysis may be conducted using a commercially available analysis apparatus known in the art and can be conducted using, for example, software such as GraphPad Prism ver. 5.4 (GraphPad Software Inc.) or JMP14 (jmp.Statistical Discovery.(TM)).

Hereinafter, the present invention will be specifically described with reference to Examples. However, the technical scope of the present invention is in no way limited to these examples.

### Examples

### [Example 1]

### (Specimen)

Study in this Example was approved (#175-4) by the ethical review board of Tokushima University and conducted at Tokushima University, LIAA Tokushima Prefecture Naruto Hospital (Tokushima University-related hospital), and National Mie Hospital from August 2012 to January 2019.

This study was conducted on infants approved by both or one of parents under sufficient informed consent among infants aged 1 year and 0 months to 6 years who were confirmed to have allergen sensitization or allergy to chicken eggs or suspected of having allergy to chicken eggs. OFC was conducted on the basis of Japanese Pediatric Guideline for Food Allergy, 2016, Food Allergy Committee, Japanese Society of Pediatric Allergy and Clinical Immunology, Kyowa Kikaku, Ltd.

A criterion for the selection of test infants will be given.
(1) An infant who was confirmed to have allergen sensitization to chicken eggs and took a chicken egg-free diet for 6 months or longer before implementation of OFC. (Except for infants who were subjected to heated egg OFC for the purpose of diagnosis within 3 months before implementation of OFC.)
(2) An infant who was confirmed to have allergen sensitization to chicken eggs, subjected to blood examination immediately before or within 4 months before implementation of OFC, and subjected to the measurement of ovomucoid-specific IgE by ImmunoCAP or the DCP method.

472 subjects were selected according to this criterion.

### (Selection of allergen)

It is known that the chicken egg component ovalbumin loses its activity as an allergen by heating. Therefore, in this study, a heated chicken egg powder was ingested in OFC, and the presence or absence of occurrence of anaphylaxis in the test infants having chicken egg allergy was tested by using, as an index, ovomucoid (OVM) which does not lose its allergenic activity by heating.

As for the 472 test infants, serum samples collected from all the test subjects from 4 weeks before OFC to the date of implementation of OFC were cryopreserved at lower than -30°C without being thawed until analysis. Before implementation of OFC, the degrees of production of an IgE antibody against ovomucoid (OVM) were measured by blood examination using ImmunoCAP (Phadia AB) generally used, and classified into classes 0 to 6. Since the present invention is aimed at verifying the usefulness of the avidity measurement of an IgE antibody in the diagnosis of the occurrence of anaphylaxis, OFC excluded negative subjects of class 0 and false positive subjects of class 1 for which avidity measurement was difficult due to low IgE antibody titers, was conducted as to 343 infants of class 2 or higher who were determined as having allergen sensitization to ovomucoid and permitted calculation of an allergen concentration that inhibited antigen-antibody binding reaction by 50% as the value of avidity.

### (Implementation of OFC)

The 343 infants of class 2 or higher determined as being allergen sensitization-positive to OVM by ImmunoCAP were subjected to OFC and thereby definitively diagnosed as to the presence or absence of chicken egg allergy, and their specimens were used in avidity measurement. The method for implementing OFC abided by the method described in Furuya K et al., Allergy. 2016; 71: 1435-1443 and Japanese Pediatric Guideline for Food Allergy, 2016, Food Allergy Committee, Japanese Society of Pediatric Allergy and Clinical Immunology, Kyowa Kikaku, Ltd.

Specifically, the test infants were grouped into 1-year-old infants and 2- to 6-year-old infants according to the amount of food ingestible depending on age. The total load of a chicken egg in OFC of the 1-year-old infants was set to 6.5 g of a heated chicken egg powder (Kewpie Corp.) (which corresponded to 1/2 of a chicken egg). The total load of a chicken egg in OFC of the 2- to 6-year-old infants was set to 13 g of a heated chicken egg powder (which corresponded to one chicken egg).

The total load was divided to 6 stages, 2/100, 4/100, 8/100, 16/100, 32/100, and 38/100, and the test was conducted by a method of allowing each test infant to ingest the heated chicken egg powder in 6 portions at 15- to 30-minute intervals by gradually increasing the load. Specifically, each 1-year-old infant ingested the heated chicken egg powder in 6 portions in the order of 0.13 g, 0.26 g, 0.52 g, 1.04 g, 2.08 g, and 2.47 g at 15- to 30-minute intervals. Each 2- to 6-year-old infant ingested the heated chicken egg powder in 6 portions in the order of 0.26 g, 0.52 g, 1.04 g, 2.08 g, 4.16 g, and 4.94 g at 15-to 30-minute intervals.

Each infant was given the heated chicken egg powder as a mixed powder supplemented with a pumpkin or sweet potato powder in order to mask the chicken egg and to correct difference in increased intake among ingestions.

When an infant who ingested the heated chicken egg powder fell into a criterion given below, this infant was definitively diagnosed as being positive to allergy to chicken eggs in OFC. When an infant did not fall into the criterion given below, this infant was definitively diagnosed as being negative to allergy to chicken eggs.

### (Criterion for definitive diagnosis of allergy positivity)

(A) Within several minutes or several hours from the start of heated chicken egg powder loading,
   one or more of
   cutaneous symptoms - itch, hives, erythema, and angioedema; and
   mucosal symptoms - conjunctival injection, nasal discharge, sneezing, and unpleasant feeling in the throat.
(B) Within several minutes or several hours from the start of heated chicken egg powder loading,
   one or more of
   respiratory symptoms - coughing and stridor;
   digestive symptoms - abdominal pain, vomiting, and diarrhea; and
   systemic symptoms - facial pallor, blood pressure reduction, and decreased level of consciousness.
(C) Within several minutes or several hours from the start of heated chicken egg powder loading,
   30% or more reduction in systolic blood pressure based on the age of a child

A subject that fell into one or more of (A), (B), and (C) described above was definitively diagnosed as being positive to allergy to chicken eggs.

### (Criterion for definitive diagnosis of occurrence of anaphylaxis)

When any one or more of the following criteria 1 to 3 was satisfied, developed anaphylaxis was determined on the basis of the description of Criteria for Diagnosing Anaphylaxis of World Allergy Organization Anaphylaxis Guidelines according to World Allergy Organ J. 2011; 4: 13-37.

1 Within several minutes or several hours from heated chicken egg powder loading, cutaneous or mucosal symptoms (e.g., systemic hives, itchiness, flush, and swelling of the lip, the tongue, or the uvula) appear, and
   one or more of the following A) and B) are satisfied:
   A) respiratory disturbance (e.g., dyspnea, expiratory stridor, inspiratory stridor, reduction in PEF (peak expiratory flow), and hypoxia), and
   B) blood pressure reduction or related organ failure (e.g., hypotension, collapse, faint, and incontinence).
2 Within several minutes or several hours from heated chicken egg powder loading, two or more of the following symptoms C) to F) appear:
   C) cutaneous or mucosal symptoms (e.g., systemic hives, itchiness, flush, and swelling of the lip, the tongue, or the uvula),
   D) respiratory disturbance (e.g., dyspnea, expiratory stridor, inspiratory stridor, reduction in PEF (peak expiratory flow), and hypoxia),
   E) blood pressure reduction or related organ failure (e.g., hypotension, collapse, faint, and incontinence), and
   F) persistent gastrointestinal symptoms (e.g., colic and vomiting).
3 Within several minutes or several hours from heated chicken egg powder loading, reduction in systolic blood pressure according to age or 30% or more reduction in systolic blood pressure was exhibited.

An infant determined as being positive in the OFC was diagnosed with chicken egg allergy, and an infant determined as being negative in the OFC was diagnosed with no chicken egg allergy. Further, among the test infants diagnosed with chicken egg allergy, an infant who manifested anaphylactic reaction was regarded as having anaphylaxis (AN+), and an infant who manifested no anaphylactic reaction was regarded as having no anaphylaxis (AN-). The classes of the test infants classified by blood examination using ImmunoCAP conducted before implementation of OFC were also described. The results are shown in Table 1.

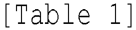

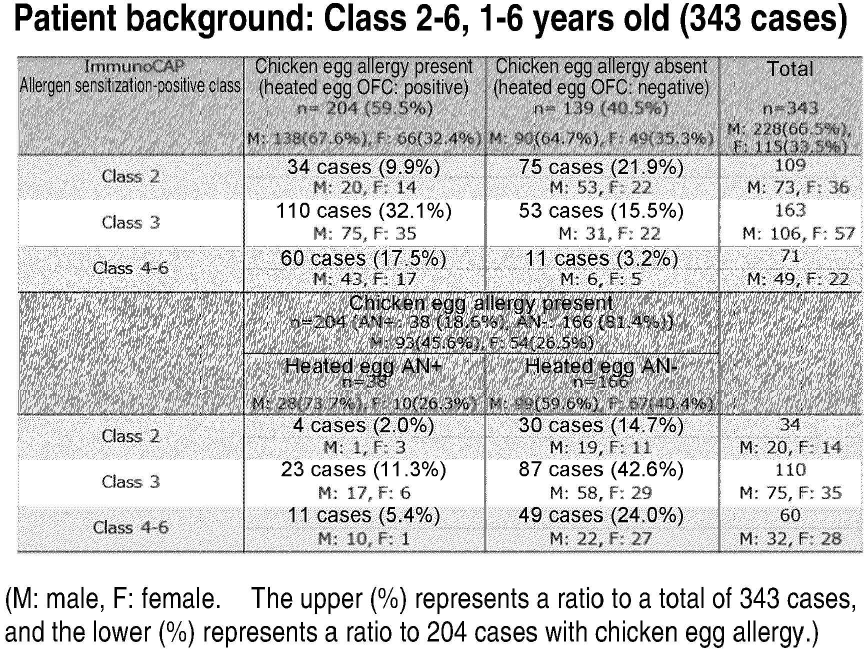

### (Results 1)

As is evident from Table 1, 204 out of the 343 1- to 6-year-old infants subjected to OFC using the heated chicken egg powder were positive in the OFC and were definitively diagnosed with chicken egg allergy. On the other hand, the remaining 139 infants were negative in the OFC and were definitively diagnosed with no chicken egg allergy. Also, 38 out of the 204 infants definitively diagnosed with chicken egg allergy were determined as developing anaphylaxis, and the remaining 166 infants were determined as developing no anaphylaxis.

Thus, 59.5% of the 1- to 6-year-old infants subjected to OFC were definitively diagnosed with chicken egg allergy, and 40.5% thereof were definitively diagnosed with no chicken egg allergy. The male-to-female ratio of the infants who were determined as being ovomucoid sensitization-positive in ImmunoCAP at the preliminary stage and classified into classes 2 to 6 was 66.5% males and 33.5% females. Thus, the male infants exhibited a significantly high value. However, in the definitive diagnosis of chicken egg allergy by OFC, no significant difference in male-to-female ratio was found in each of the groups with chicken egg allergy, without chicken egg allergy, with chicken egg allergy and AN+, and with chicken egg allergy and AN-. Among the infants definitively diagnosed with chicken egg allergy, AN+ infants who manifested serious symptoms accounted for 18.6%. The allergic symptoms of AN-infants definitively diagnosed with chicken egg allergy were not in the state of life-threatening hypersensitive reaction.

### (Results 2)

Table 2 shows data on only 1- to 3-year-old test infants for which the diagnosis of allergy is difficult in common medical examination due to non-fully developed immune system among the test infants.

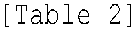

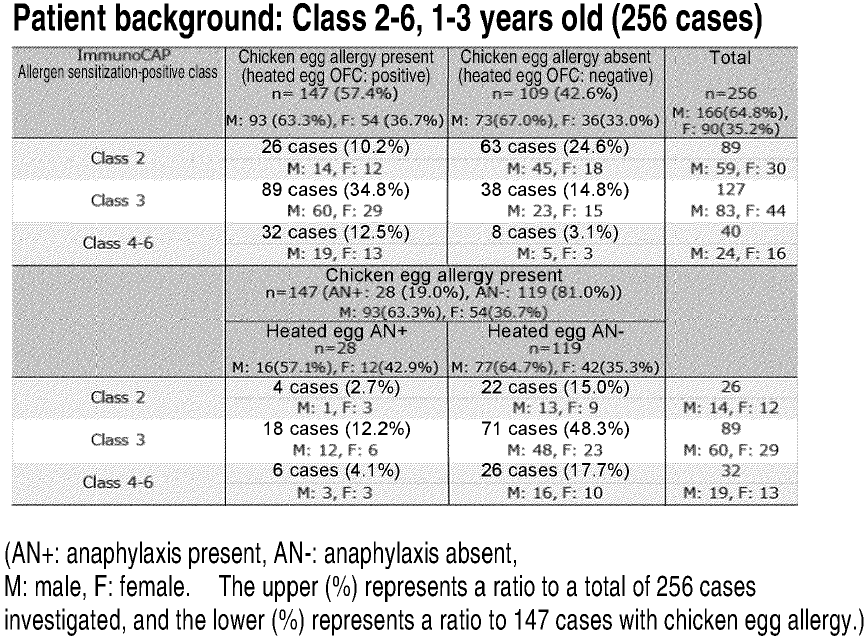

As is evident from Table 2, 147 out of the 256 1- to 3-year-old infants subjected to OFC using the heated chicken egg powder were positive in the OFC and were definitively diagnosed with chicken egg allergy. On the other hand, the remaining 109 infants were negative in the OFC and were definitively diagnosed with no chicken egg allergy. Also, 28 out of the 147 infants definitively diagnosed with chicken egg allergy were determined as developing anaphylaxis, and the remaining 119 infants were determined as developing no anaphylaxis.

Thus, 57.4% of the 1- to 3-year-old infants subjected to OFC were definitively diagnosed with chicken egg allergy, and 42.6% thereof were definitively diagnosed with no chicken egg allergy. Among the infants definitively diagnosed with chicken egg allergy, AN+ infants who manifested serious symptoms accounted for 19.0%. The allergic symptoms of AN- infants definitively diagnosed with chicken egg allergy were not in the state of life-threatening hypersensitive reaction. Thus, the incidence of anaphylaxis in infants definitively diagnosed with chicken egg allergy among 1- to 3-year-old infants reportedly having non-fully developed immune system was confirmed to have no significant difference from that in 1-to 6-year-old infants.

### [Example 2]

### [Measurement of OVM-specific IgE antibody titer by DCP method]

Serum samples collected from the 343 1- to 6-year-old infants before OFC were used. The antigenic protein OVM (manufactured by Sigma-Aldrich Co. LLC) known as an allergen having a definitive molecular weight, contained in chicken eggs was immobilized as an antigen on a DCP chip, and the amount of an IgE antibody bound to OVM was measured as an OVM-specific IgE antibody titer. For the quantification of the IgE antibody titer, a fluorescence intensity calibration curve was created from human IgE antibody standards (75/502) having known concentrations, purchased from National Institute for Biological Standards and Control (Hertfordshire, England), and the OVM-specific IgE antibody titer (BUe/mL) was measured using the DCP chip.

### [Preparation of OVM-immobilized DCP chip]

### (Activation of chip)

An amino group-containing electrostatic layer was provided on a DCP chip made of a glass substrate, and a negatively charged carboxyl group was further introduced thereto with polyacrylic acid. The resulting substrate was activated with shaking at room temperature for 30 minutes in the dark in a chemical cross-linking agent (100 mM WSC•HCl, 100 mM NHS, 0.1 M potassium phosphate buffer solution (pH 6.0)). After discarding of the chemical cross-linking agent after reaction, the substrate was washed with MilliQ water twice each for 1 minute with shaking. Immediately thereafter, water was removed using a desktop centrifuge (Allegra(TM) X-22R Centrifuger, manufactured by Beckman Coulter, Inc.) to prepare an activated chip.

### (Coupling reaction of OVM)

The antigenic protein OVM (manufactured by Sigma-Aldrich Co. LLC) was dissolved at a concentration of 0.2 to 2.0 mg/mL in a solution supplemented with 5 to 30% DMSO or 5 to 30% PEG300 to prepare an antigenic protein solution. The antigenic protein solution thus prepared was aliquoted to a 384-well plate with flat bottom (manufactured by Corning Inc.), and 4 nL thereof was spotted on the activated chip using a microarrayer (OmniGrid Accent, manufactured by DIGILAB) and then dried at 15°C to 30°C for 1 to 18 hours to immobilize the OVM antigenic protein. Figure 2 shows a schematic view of the OVM-immobilized DCP chip.

### (Blocking reaction of unreacted active group)

The blocking reagent Blockmaster (JSR Corp.) was added to the antigenic protein OVM-immobilized chip in the reaction plate (wells), and the chip was left standing under refrigeration (4°C) in the dark for overnight reaction. After removal of the blocking reagent by suction using an aspirator (VARIABLE SPEED PUMP, manufactured by Bio-Rad Laboratories, Inc.), the chip was transferred to the reaction plate again, and 10 mL of a washing liquid (50 mM TTBS) was added thereto, followed by shaking for 5 minutes and subsequent removal of the washing liquid by suction using the aspirator. The plate was washed 3 times in the same manner as above and then further washed with purified water (MilliQ water) 3 times. Water droplets on the surface of the chip were removed by centrifugation (at 2000 rpm for 1 minute) using a centrifuge (Allegra(TM), X-22R Centrifuge (manufactured by Beckman Coulter, Inc.)) to prepare an OVM-immobilized DCP chip.

### (Primary antibody reaction)

The serum sample of each test infant was appropriately diluted with a sample diluent (20 mM phosphate buffer, pH 7.4/0.3 M KCl/0.05% Tween 20) to prepare a diluted serum solution (primary antibody solution). 10 µL of this diluted sample was added to each reaction well and left standing at 37°C for 2 hours in the dark.

### (Reaction with secondary antibody)

The diluted sample thus obtained by the operation was removed by suction using an aspirator (VARIABLE SPEED PUMP, manufactured by Bio-Rad Laboratories, Inc.). Then, the chip was transferred to a case for washing, and 10 mL of a washing liquid (TTBS: 50 mM Tris-HCl, pH 7.5, 150 mM NaCl, 0.95% Tween 20) was added thereto. Then, the chip was washed by a 5-minute washing operation repeated 3 times using Double-Shaker NR3, and further washed 3 times each for 1 minute by the addition of purified water (MilliQ water). Water droplets on the surface of the chip were removed by centrifugation (at 2000 rpm for 1 minute) using the centrifuge. Subsequently, a fluorescently labeled secondary antibody (HiLyte Fluor(TM) or 555 conjugated anti human IgE (HyTest Ltd.) (diluent: IMMUNO SHOT Platimun/1% BSA, final diluted concentration: 10 µg/mL)) was prepared into a secondary antibody solution. 10 µL of the secondary antibody solution was aliquoted into each reaction well on the slide and left standing at 37°C for 2 hours in the dark.

### (Detection of primary antibody captured by antigen)

After removal of the diluted secondary antibody solution by suction using an aspirator, the chip was put in a case for washing, and a 5-minute washing operation was repeated 3 times using Double-Shaker NR3 (manufactured by TAITECH Corp.). Then, the chip was rinsed 3 times each for 1 minute by the addition of purified water (MilliQ water) and dried by removing water droplets using the centrifuge. The fluorescence intensity was measured (Ex: 532 nm, Em: 570 nm) with a fluorescent scanner (3D Gene Scanner, manufactured by Toray Industries, Inc.) to numerically convert the fluorescence intensity of the spots obtained from each chip. For the unit of measurement, the antibody titer of an IgE antibody bound to an antigen through antigen-antibody reaction was indicated by binding unit (BUe) and expressed by measurement from a calibration curve of the fluorescence intensity of antibody standards with known concentrations immobilized on a chip. In the following description, 1 BUe = 2.4 ng.

### [Example 3]

### [Measurement of avidity of OVM-specific IgE antibody]

The avidity of an OVM-specific IgE antibody against OVM was measured using serum samples collected from the 343 1- to 6-year-old infants before OFC and the antigenic protein OVM (manufactured by Sigma-Aldrich Co. LLC).

The following reaction was performed using the OVM-immobilized DCP chip.

### (Primary antibody reaction)

The serum sample of each test infant was appropriately diluted, for example, 2.5-fold, with a sample diluent (20 mM phosphate buffer, pH 7.4/0.3 M KCl/0.05% Tween 20) to prepare a diluted serum solution (primary antibody solution). The antibody titer was measured. Subsequently, the obtained antibody titer of the diluted solution was readjusted to a range in which competitive binding inhibitory activity (avidity) depending on the amount of a soluble antigen added was detectable. Specifically, the adjustment was made such that the lower limit value of fluorescence intensity exhibited by OVM IgE was > measurement limit value (blank + 5SD) and the upper limit value of the fluorescence intensity fell within the range of 5,000 or less. Six types of OVM solutions adjusted with the sample diluent to 6 concentrations, 0, 0.2, 2.0, 20, 100, and 200 nM, were prepared as competitive binding inhibitors. To determine IC50 values, preliminary reaction was performed in advance by varying concentrations of soluble OVM by adding 6 µL each of the 6 types of OVM solutions to the diluted serum solution (6 µL) (a total of 12 µL), followed by reaction at 25°C for 30 minutes before measuring the antigen-antibody reaction between an IgE antibody contained in each specimen and the OVM antigen immobilized on the chip. 10 µL each of the reaction solutions that have undergone the preliminary reaction was collected, added to the OVM-immobilized DCP chip, and left standing at 37°C for 2 hours in the dark. The blank + 5SD represents the lower limit value of the measurement limit. Specifically, fluorescence intensity derived from the nonspecific binding of a fluorescently labeled secondary antibody contained in a reaction solution to an antigen under conditions of a buffer solution alone without specimen serum was defined as a blank value. This fluorescence intensity was repetitively measured 5 times, and a standard deviation (SD) value of fluorescence intensity was calculated. Fluorescence intensity equal to or higher than the blank + 5SD (lower limit value of the measurement limit) was regarded as the fluorescence intensity of IgE derived from the sample.

### (Reaction with secondary antibody)

The diluted serum solution thus reacted on the DCP chip was removed by suction using an aspirator (VARIABLE SPEED PUMP, manufactured by Bio-Rad Laboratories, Inc.). Then, the chip was transferred to a case for washing, and 10 mL of a washing liquid (50 mM TTBS) was added thereto. Then, the chip was washed by a 5-minute washing operation repeated 3 times using Double-Shaker NR3, and further washed 3 times each for 1 minute by the addition of purified water (MilliQ water). Water droplets on the surface of the chip were removed by centrifugation (at 2000 rpm for 1 minute) using the centrifuge. Subsequently, a fluorescently labeled secondary antibody (HiLyte Fluor(TM) or 555 conjugated anti human IgE (HyTest Ltd.) (diluent: IMMUNO SHOT Platimun/1% BSA, final diluted concentration: 10 µg/mL)) was prepared. 10 µL of the secondary antibody solution was aliquoted into each reaction well on the slide and left standing at 37°C for 2 hours in the dark.

After removal by suction using an aspirator, the chip was transferred to a case for washing, and 10 mL of a washing liquid (50 mM TTBS) was added thereto. Then, the chip was washed by a 5-minute washing operation repeated 3 times using Double-Shaker NR3, and further washed 3 times each for 1 minute by the addition of purified water (MilliQ water). Water droplets on the surface of the chip were removed by centrifugation (at 2000 rpm for 1 minute) using the centrifuge. The amount of residual fluorescence was measured as fluorescence intensity (Ex: 532 nm, Em: 570 nm) with a fluorescent scanner (3D Gene Scanner, manufactured by Toray Industries, Inc.) to numerically convert the fluorescence intensity of the spots obtained from each chip. For each test infant or child, an OVM concentration that exhibited 50% fluorescence intensity was indicated as an "IC50" value when fluorescence intensity from an OVM-free solution, i.e., a competitive binding inhibitor concentration of 0, was defined as 100%.

### [ROC analysis]

### [Reference Example 1]

ROC analysis was conducted on the 1- to 6-year-old infants (343 cases) subjected to OFC in [Example 1]. On the basis of the results of diagnosing a subject positive in OFC with chicken egg allergy and diagnosing a subject negative in OFC with no chicken egg allergy, the ROC analysis was conducted using the OVM-specific IgE titer and the avidity of OVM-specific IgE measured by the DCP method, and the OVM-specific IgE titer measured by ImmunoCAP, as to five types of parameters:
(1) a value obtained by multiplying the value of 1/IC50 by the OVM-specific IgE titer measured by the DCP method [DCP OVM sIgE/DCP OVMs IgE IC50 [(BUe/mL)/(nM)]];
(2) the IC50 value of OVM-specific IgE measured by the DCP method (DCP OVM sIgE IC50 (nM));
(3) the value of 1/IC50 of OVM-specific IgE measured by the DCP method (1/DCP OVMs IgE IC50 (nM));
(4) the OVM-specific IgE titer measured by the DCP method (DCP OVMs IgE (BUe/mL)); and
(5) the OVM-specific IgE titer measured by ImmunoCAP (CAP OVMs IgE (UA/mL)).

The results are shown in Figure 3. Hereinafter, the ROC analysis was conducted using GraphPad Prism ver. 5.4 (GraphPad Software Inc.) or JMP14 (jmp.Statistical Discovery.(TM)).

The ROC analysis targeting the 343 1- to 6-year-old infants was conducted for each of the five types of parameters as to the test subjects definitively diagnosed as allergy-positive subjects and the test subjects definitively diagnosed as allergy-negative subjects depending on the definitive diagnosis of allergy to OVM by OFC. An AUC value, a cutoff value, sensitivity, and specificity were calculated. The results are shown in Table 3.

**[Table 3]**

| Variable name (parameter) | | **AUC** | Lower limit **95%** | Upper limit **95%** | Cutoff value | Sensitivity | Specificity |
|---|---|---|---|---|---|---|---|
| DCP OVM sIgE/DCP OVM sIgE IC50 [(BUe/mL)/(nM)] | ① | 0.79 | 0.74 | 0.84 | 4459.00 | 0.61 | 0.83 |
| DCP OVM sIgE IC50 (nM) | ② | 0.72 | 0.67 | 0.77 | 1.3 | 0.66 | 0.71 |
| 1/DCP OVM sIgE IC50 (nM) | ③ | 0.72 | 0.66 | 0.77 | 0.77 | 0.66 | 0.71 |
| DCP OVM sIgE (BUe/mL) | ④ | 0.75 | 0.70 | 0.80 | 4283.24 | 0.69 | 0.75 |
| CAP OVM sIgE (U_{A}/mL) | ⑤ | 0.76 | 0.70 | 0.81 | 7.24 | 0.66 | 0.79 |

| | | | | | | | |
|---|---|---|---|---|---|---|---|
| ①, ②, ③, ④, and ⑤ correspond to parameters described in Figure 3. | | | | | | | |

The AUC value of the measured OVM-specific IgE titer was almost equivalent between the CAP OVM sIgE method (UA/mL) and the DCP OVM sIgE method (BUe/mL). Among the five types of parameters studied, "DCP OVM sIgE/DCP OVM sIgE IC50" obtained by multiplying the IgE antibody titer by the OVM avidity had the highest AUC value of 0.79. In this respect, the cutoff value of definitive allergy diagnosis exhibited 4459.00 (BUe/mL)/(nM). Thus, when the value of DCP OVM sIgE/DCP OVM sIgE IC50 obtained by multiplying the IgE antibody titer by the OVM avidity is equal to or higher than this cutoff value, i.e., 4459.00 (BUe/mL)/(nM), a high risk of chicken egg allergy can be determined. When this value is less than the cutoff value, a low risk of chicken egg allergy can be determined. Thus, this parameter was expected to contribute to improvement in quality of life of patients in such a way that diet restriction carried out so far without exception on patients suspected of having chicken egg allergy is carried out on only high-risk patients while the diet restriction of low-risk patients is canceled. As shown in the lower part of Figure 3, the AUC value of DCP OVM sIgE/DCP OVM sIgE IC50 (quantity × quality) obtained by multiplying the quantified IgE value by the OVM avidity, as compared with the single parameter such as the IC50 value or the OVM avidity, exhibited a P value which indicated significant difference, showing that this parameter can accurately determine the risk of chicken egg allergy and the absence of chicken egg allergy. In the specification of the present application, "OVM sIgE" means ovomucoid-specific IgE or IgE specific for ovomucoid.

### [Reference Example 2]

Figure 4 shows results of conducting ROC analysis targeting, particularly, 256 test subjects (1 to 3 years old) for which the diagnosis of allergy is reportedly difficult in common medical examination due to non-fully developed immune system among the test subjects (1 to 6 years old) shown in [Example 1], using the five types of parameters as to test subjects definitively diagnosed as allergy-positive subjects and test subjects definitively diagnosed as allergy-negative subjects depending on the definitive diagnosis of allergy to OVM by OFC.

The ROC analysis targeting the 256 1- to 3-year-old infants was conducted as to the test subjects definitively diagnosed as allergy-positive subjects and the test subjects definitively diagnosed as allergy-negative subjects depending on the definitive diagnosis of allergy by OFC. An AUC value, a cutoff value, sensitivity, and specificity were calculated for each of the five types of parameters. The results are shown in Table 4.

**[Table 4]**

| Variable name (parameter) | | **AUC** | Lower limit **95%** | Upper limit **95%** | Cutoff value | Sensitivity | Specificity |
|---|---|---|---|---|---|---|---|
| DCP OVM sIgE/DCP OVM sIgE IC50 [(BUe/mL)/(nM)] | ① | 0.79 | 0.73 | 0.84 | 1556.70 | 0.73 | 0.71 |
| DCP OVM sIgE IC50 (nM) | ② | 0.70. | 0.64 | 0.76 | 1.13 | 0.57 | 0.76 |
| 1/DCP OVM sIgE IC50 (nM) | ③ | 0.70 | 0.64 | 0.76 | 0.77 | 0.59 | 0.74 |
| DCP OVM sIgE (BUe/mL) | ④ | 0.75 | 0.69 | 0.81 | 3587.27 | 0.72 | 0.72 |
| CAP OVM sIgE (U_{A}/mL) | ⑤ | 0.75 | 0.68 | 0.81 | 6.49 | 0.65 | 0.78 |

| | | | | | | | |
|---|---|---|---|---|---|---|---|
| ①, ②, ③, ④, and ⑤ correspond to parameters described in Figure 4. | | | | | | | |

### (Results)

The test subjects (1 to 3 years old) for which the diagnosis of allergy is reportedly difficult were also studied using the five types of parameters in the same manner as in the test subjects (1 to 6 years old). For the test subjects (1 to 3 years old), the AUC values of the OVM-specific IgE titers (BUe/mL) measured by ImmunoCAP (UA/mL) and the DCP method were confirmed to be almost equivalent to the values for the test subjects (1 to 6 years old). Among the five types of parameters studied, DCP OVM sIgE/DCP OVM sIgE IC50 obtained by multiplying the DCP OVM sIgE value by the avidity had the highest AUC value of 0.79 as compared with the other parameters. In this respect, the cutoff value of definitive allergy diagnosis exhibited 1556.70 (BUe/mL) / (nM) and was thus a value as low as 35% of the cutoff value for the test subjects (1 to 6 years old). As shown in the lower part of Figure 4, for the test subjects (1 to 3 years old), the AUC value of DCP OVM sIgE/DCP OVM sIgE IC50 (quantity × quality) obtained by multiplying the quantified IgE value by the OVM avidity, as compared with the single parameter such as the IC50 value or the OVM avidity, exhibited a P value which indicated significant difference, as in the test subjects (1 to 6 years old), showing that this parameter can accurately determine the risk of chicken egg allergy and the absence of chicken egg allergy.

### [Example 4]

Five types of parameters, (A) CAP OVM sIgE (UA/mL), (B) DCP OVM sIgE (BUe/mL), (C) DCP OVM sIgE/DCP OVM sIgE IC50 [(BUe/mL)/(nM)], (D) DCP OVM sIgE IC50 (nM), and (E) 1/DCP OVM sIgE IC50 (nM), were evaluated for their diagnostic accuracy and usefulness, as shown in Figure 5, as to the diagnosis of a risk of occurrence of anaphylaxis by OFC in the 343 1- to 6-year-old chicken egg allergen sensitization-positive infants shown in Table 1. A significant difference test was conducted among the groups. The statistical data of Figure 5 is shown in Table 5 below.

### (Results)

As is evident from Figure 5 and Table 5, (A) CAP OVM sIgE (UA/mL) or (B) DCP OVM sIgE (BUe/mL) of the conventional method for quantifying allergen-specific IgE had no detectable significant difference between groups in the diagnosis of the "state of life-threatening hypersensitive reaction or the occurrence of anaphylaxis", i.e., [(AN+) and (AN-)], though the presence (EA+) or absence (EA-) of chicken egg allergy was able to be diagnosed with significant difference. However, it was revealed that use of (C) DCP OVM sIgE/DCP OVM sIgE IC50 [(BUe/mL)/(nM)] as a parameter permitted differentiation between (AN+) and (AN-) with significant difference (P = 0.0041) among the chicken egg allergy-positive subjects. The values of (D) DCP OVM sIgE IC50 (nM) and (E) 1/DCP OVM sIgE IC50 (nM) also exhibited relatively favorable P values of 0.0183 and 0.0184, respectively. Thus, use of the OVM avidity increased diagnostic accuracy, particularly, in the diagnosis of a risk of occurrence of anaphylaxis with its effect most evidently shown by the parameter DCP OVM sIgE/DCP OVM sIgE IC50 [(BUe/mL)/(nM)].

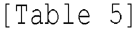

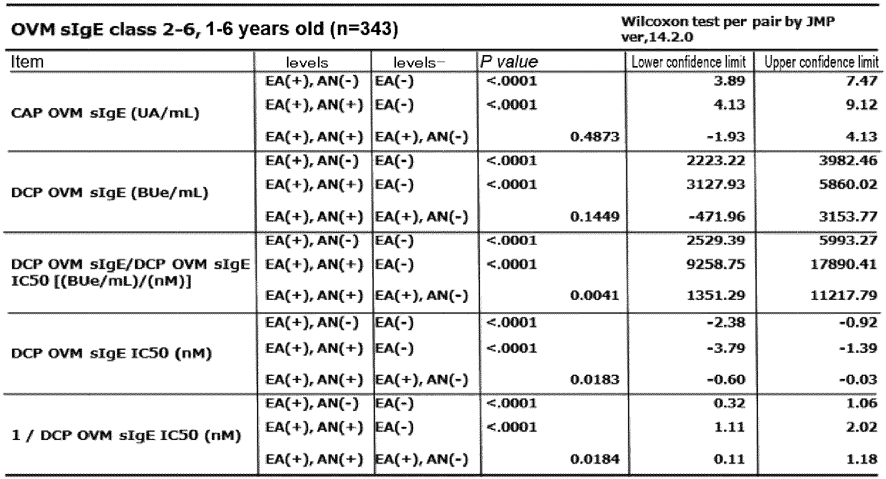

Figure 6 shows results of conducting ROC analysis using the five types of parameters as to the diagnosis of the occurrence of anaphylaxis to OVM on the basis of the OFC results about the 1- to 6-year-old test subjects (343 cases).

An AUC value, a cutoff value, sensitivity, and specificity targeting the 343 1- to 6-year-old infants subjected to the ROC analysis were calculated for each of the five types of parameters as to test subjects diagnosed as anaphylaxis cases and test subjects diagnosed as non-anaphylaxis cases depending on the diagnosis of the occurrence of anaphylaxis by OFC. The results are shown in Table 6.

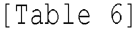

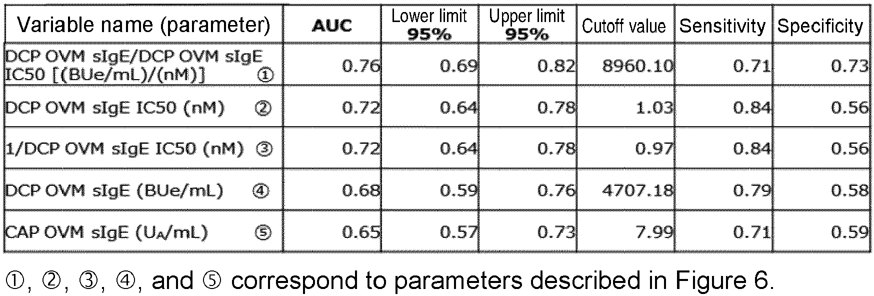

In the ROC analysis, use of the parameter DCP OVM sIgE/DCP OVM sIgE IC50 [(BUe/mL) / (nM)] obtained by multiplication by the OVM avidity, as compared with the other parameters, exhibited the highest AUC of 0.76, which fell within the range of 0.7 to 0.8. Therefore, favorable diagnostic accuracy was determined for the occurrence of anaphylaxis to chicken eggs. The cutoff value of this parameter exhibited 8960.10 [(BUe/mL)/(nM)], which was approximately 2 times higher than 4459.00 [(BUe/mL)/(nM)] for the risk of chicken egg allergy. A value exceeding this value was able to be expected to indicate a higher risk of occurrence of anaphylaxis to chicken eggs. As shown in the lower part of Figure 6, for the test subjects (1 to 6 years old), the AUC value of DCP OVM sIgE/DCP OVM sIgE IC50 (quantity × quality) obtained by multiplying the quantified IgE value by the OVM avidity, as compared with the single parameter of the quantified IgE value [(UA/mL)/mL] of the conventional method, exhibited a P value which indicated significant difference, showing that this parameter can accurately determine the risk of chicken egg allergy and the absence of chicken egg allergy.

### [Example 5]

Five types of parameters, (A) CAP OVM sIgE (UA/mL), (B) DCP OVM sIgE (BUe/mL), (C) DCP OVM sIgE/DCP OVM sIgE IC50 [(BUe/mL) / (nM)], (D) DCP OVM sIgE IC50 (nM), and (E) 1/DCP OVM sIgE IC50 (nM), were evaluated for their diagnostic accuracy and usefulness as to the diagnosis of a risk of occurrence of anaphylaxis by OFC in the 256 1- to 3-year-old infants shown in Table 2 for which the diagnosis of allergy was difficult due to non-fully developed immune system. A significant difference test was conducted among the groups. The results are shown in Figure 7. The statistical data of Figure 7 is shown in Table 7 below.

### (Results)

As is evident from Figure 7, (A) CAP OVM sIgE (UA/mL) or (B) DCP OVM sIgE (BUe/mL) exhibited no significant difference between groups, as in the 1- to 6-year-old subjects, in the diagnosis of a risk of occurrence of anaphylaxis, though the presence (EA+) or absence (EA-) of chicken egg allergy was able to be diagnosed with significant difference. However, it was revealed that use of (C) DCP OVM sIgE/DCP OVM sIgE IC50 [(BUe/mL)/(nM)] as a parameter permitted differentiation between (AN+) and (AN-) with significant difference (P = 0.0077) among the chicken egg allergy-positive subjects. (D) DCP OVM sIgE IC50 (nM) and (E) 1/DCP OVM sIgE IC50 (nM) serving as antigen affinity parameters also exhibited favorable P values of 0.0092 and 0.0093, respectively, revealing that these parameters are useful in the predictive diagnosis of the occurrence of anaphylaxis.

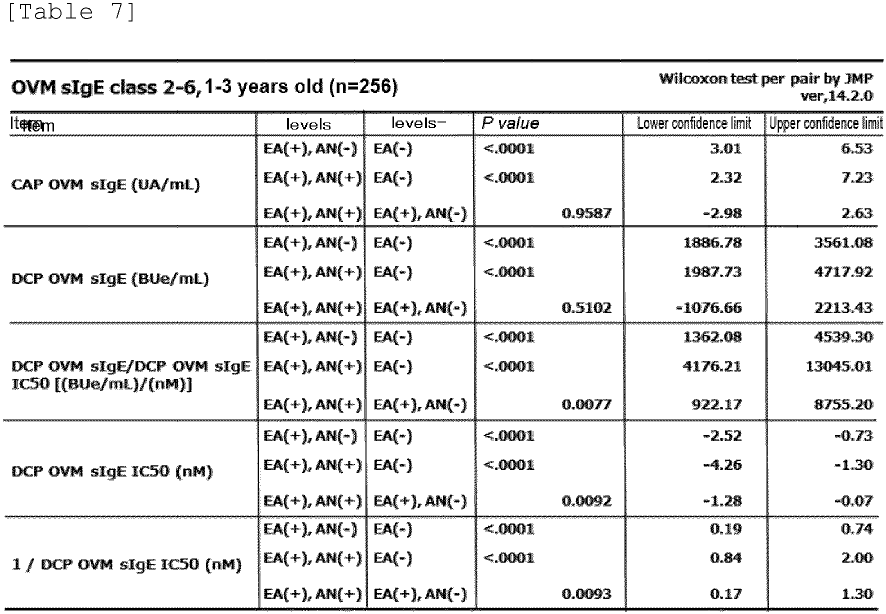

An AUC value, a cutoff value, sensitivity, and specificity targeting the 256 1- to 3-year-old infants subjected to the ROC analysis were calculated for each of the five types of parameters as to test subjects diagnosed as anaphylaxis cases and test subjects diagnosed as non-anaphylaxis cases depending on the diagnosis of the occurrence of anaphylaxis by OFC. The results are shown in Figure 8 and Table 8.

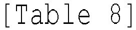

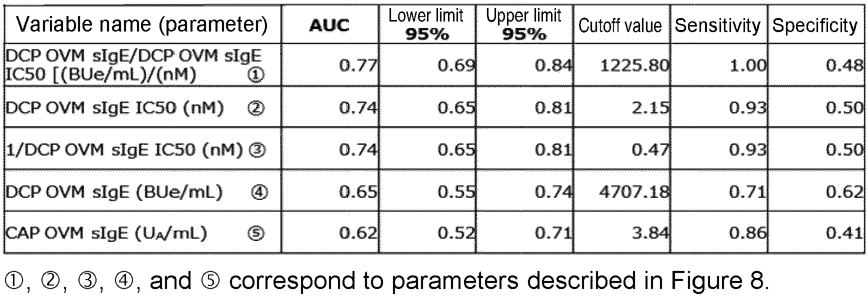

In the ROC analysis between subjects with anaphylaxis and subjects without anaphylaxis of the 1- to 3-year-old infants for which the occurrence of anaphylaxis had heretofore been difficult to diagnose, the cutoff value of DCP OVM sIgE/DCP OVM sIgE IC50 [(BUe/mL)/(nM)] was 1225.80 as shown in Table 8 and was thus a value as low as 13.7% of the cutoff value (8960.10) exhibited for the 1- to 6-year-old subjects. It thus became clear that the 1- to 3-year-old infants were susceptible to anaphylaxis. The parameter (quantity × quality) obtained by multiplying the DCP OVM sIgE value by the OVM avidity, as shown in Figure 8 and Table 8, exhibited the highest AUC value of 0.77 on the ROC curve among the 5 groups studied. In this respect, its sensitivity exhibited the highest value of 1.00, showing that this parameter is useful in the diagnosis of a risk of occurrence of anaphylaxis. In these results, (C) DCP OVM sIgE/DCP OVM sIgE IC50 [(BUe/mL)/(nM)] shown in Figure 7 and Table 7 exhibited a P value of P = 0.0077 with significant difference (P < 0.008) between the EA(+) (AN-) group and the EA(+)(AN+) group. (D) DCP OVM sIgE IC50 (nM) and (E) 1/DCP OVM sIgE IC50 (nM) also exhibited P values of P = 0.0092 and P = 0.0093, respectively, between the EA(+) (AN-) group and the EA(+) (AN+) group and thus had a relatively favorable outcome. As shown in the lower part of Figure 8, for the test subjects (1 to 3 years old), the AUC value of DCP OVM sIgE/DCP OVM sIgE IC50 (quantity × quality) obtained by multiplying the quantified IgE value by the OVM avidity, as compared with the single parameter of the IgE antibody titer (UA/mL) or (BUe/mL) of the conventional method, also exhibited significant difference, showing that this parameter can accurately determine the risk of chicken egg anaphylaxis and the absence of chicken egg anaphylaxis. Thus, it was expected that when DCP OVM sIgE/DCP OVM sIgE IC50 [(BUe/mL)/(nM)] is equal to or higher than this cutoff value of 1225.80 in the diagnosis of a risk of occurrence of anaphylaxis in 1- to 3-year-old subjects, a high risk of occurrence of anaphylaxis to chicken eggs can be determined.

### (Conclusion)

The previous diagnosis of allergy involves measuring only the (quantity) of allergen-specific IgE in blood as shown in Figure 1, and antigen affinity as the quality of allergen-specific IgE is not taken into consideration. Since the reaction of an antibody consists of the "quantity" and "quality" of the antibody, the bioreaction of allergen-specific IgE should be evaluated according to its "quantity" and "quality". In the present invention, the usefulness of antigen avidity was studied as the "quality" of the antibody.

The avidity of IgE against an allergen can be indicated by 1/IC50 value by measuring the competitive binding inhibitory activity (IC50 value) between an immobilized allergen and a soluble allergen. This method has the advantages that not only can evaluation be made even in the presence of an IgG, IgA, IgD, and/or IgM antibody that recognizes the same antigen as that of IgE but difference in affinity among allergens or difference in mutual affinity between different allergens can be evaluated with the same parameter. In the present invention, it was confirmed that a risk of occurrence of anaphylaxis which is difficult to diagnose by a conventional method for quantifying allergen-specific IgE, can be predictively diagnosed by measuring avidity (1/IC50 value) of a polyclonal IgE antibody against an allergen in human serum, and using the parameter DCP OVM sIgE/DCP OVM sIgE IC50 [(BUe/mL)/(nM)] obtained by multiplying the antibody titer of DCP OVM sIgE (BUe/mL) by the avidity. Further, it was newly revealed that in the diagnosis of a risk of occurrence of anaphylaxis in 1- to 6-year-old and 1- to 3-year-old chicken egg allergy-positive patients, the values of DCP OVM sIgE IC50 (nM) and 1/DCP OVM sIgE IC50 (nM) serving as single parameters for avidity against an allergen can also discriminate the presence or absence of occurrence of anaphylaxis with significant difference, though the degree of the significant difference is decreased as compared with DCP OVM sIgE/DCP OVM sIgE IC50 [(BUe/mL)/(nM)] obtained by multiplying the antibody titer of DCP OVM sIgE(BUe/mL) by the avidity.

### [Example 6]

In order to examine the implementability (effectiveness) of the cutoff values of the IC50 values of chicken egg allergy and chicken egg anaphylaxis calculated from previous ROC analysis and the criterion [(BUe/mL)/IC50] created on the basis of an IgE antibody titer and avidity of an IgE antibody against an allergen, Takatsuki General Hospital (Osaka, Japan), a hospital located in a region different from that of the hospital at which the preceding data (Tables 1 to 8, etc.) was obtained, was requested to recruit allergy patients as OFC participants from February 20, 2020 under the approval of the ethical review board of Takatsuki General Hospital (ethical review board approval No: 2019-92). Blood was collected under the consents of the patients and their families, and the measurement of OVM-specific IgE values and the calculation of avidity values thereof were carried out.

At the first stage of measurement, 15 out of 19 cases of chicken egg allergy patients from which data was able to be collected for approximately 4 months from February 20, 2020 to June 17, 2020 were subjected to heated chicken egg OFC involving OVM. The remaining 4 chicken egg allergy patients were excluded from this study because ovalbumin, an allergen of raw eggs, was found to be responsible for their allergy. The effectiveness of the cutoff values of the IC50 values of chicken egg allergy and chicken egg anaphylaxis described in the patent and the [(BUe/mL)/IC50] value was verified as to the 15 cases subjected to heated chicken egg OFC.

Two out of the 15 cases that were able to undergo heated chicken egg OFC at the pediatrics department of Takatsuki General Hospital were 1- to 3-year-old subjects, and the remaining 13 cases were 8- to 15-year-old subjects. In this study, the 13 cases of 8- to 15-year-old subjects were studied for effectiveness using the cutoff value for the 1- to 6-year-old subjects.

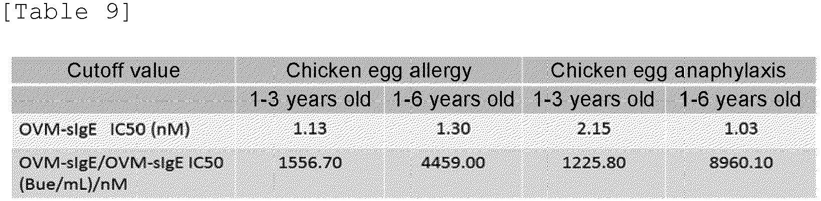

### (Cutoff value in chicken egg allergy diagnosis)

The contents of the numerical values shown in Table 9 above are as follows.
[1] Cutoff values of results of conducting ROC analysis as to test subjects definitively diagnosed as chicken egg allergy-positive subjects and test subjects definitively diagnosed as chicken egg allergy-negative subjects depending on the definitive diagnosis of allergy to OVM by OFC in 1- to 3-year-old and 1- to 6-year-old infants were extracted from Tables 3 and 4 above as follows:
   (1) A cutoff value regarding the presence or absence of allergy in the IC50 value of OVM-specific IgE (OVM sIgE IC50 (nM)) measured by the DCP method;
      (i.e., a 1- to 3-year-old subject having a value of less than 1.13 nM or a 1- to 6-year-old having a value of less than 1.30 nM can be determined as being allergy-positive.)
   (2) A cutoff value regarding the presence or absence of allergy in [OVM sIgE/OVMs IgE IC50 [(BUe/mL)/(nM)]] obtained by multiplying the OVM-specific IgE titer measured by the DCP method by the value of 1/IC50;
      (a 1- to 3-year-old subject having a value equal to or more than 1556.70 (BUe/mL)/(nM) or a 1- to 6-year-old having a value equal to or more than 4459.00 (BUe/mL)/(nM) can be determined as being allergy-positive.)

### (Cutoff value in diagnosis of occurrence of chicken egg anaphylaxis)

[2] Cutoff values of results of conducting ROC analysis as to test subjects diagnosed as anaphylaxis cases and test subjects diagnosed as non-anaphylaxis cases depending on the diagnosis of the occurrence of chicken egg anaphylaxis by OFC in 1- to 3-year-old and 1- to 6-year-old infants were extracted from Tables 6 and 8 above as follows:
(3) A cutoff value regarding the presence or absence of occurrence of anaphylaxis in the IC50 value of OVM-specific IgE (OVM sIgE IC50 (nM)) measured by the DCP method;
(i.e., a 1- to 3-year-old subject having a value of less than 2.15 nM or a 1- to 6-year-old having a value of less than 1.03 nM can be determined as having the possibility of occurrence of anaphylaxis.)
(4) A cutoff value regarding the presence or absence of occurrence of anaphylaxis in [OVM sIgE/OVMs IgE IC50 [(BUe/mL)/(nM)]] obtained by multiplying the OVM-specific IgE titer measured by the DCP method by the value of 1/IC50;
(a 1- to 3-year-old subject having a value equal to or more than 1225.80 (BUe/mL)/(nM) or a 1- to 6-year-old having a value equal to or more than 8960.00 (BUe/mL)/(nM) can be determined as having the possibility of occurrence of anaphylaxis.)

Table 10 below shows results about 3 parameters: DCP OVM-sIgE, DCP OVM-sIgE IC50, and DCP OVM-sIgE/DCP OVM-sIgE IC50 obtained from the two 1- to 3-year-old cases and the 13 8- to 15-year-old cases among the cases subjected to OFC at Takatsuki General Hospital.

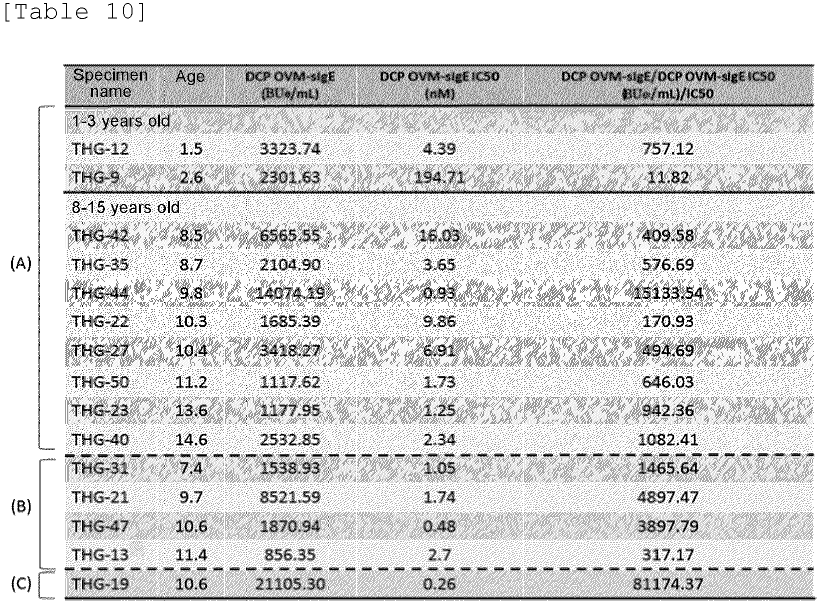

(A) of Table 10 is a group that was determined as being chicken egg allergy-negative as a result of heated chicken egg OFC (chicken egg allergy-negative/anaphylaxis occurrence-negative group), and the two 1- to 3-year-old infants and eight 8- to 15-year-old children belonged thereto. (B) is a group that was determined as being chicken egg allergy-positive as a result of heated chicken egg OFC and developed no anaphylaxis (chicken egg allergy-positive/anaphylaxis occurrence-negative group). (C) is a group that was determined as being chicken egg allergy-positive as a result of heated chicken egg OFC and developed anaphylaxis (group with chicken egg anaphylaxis).

### (Chicken egg allergy-negative/anaphylaxis occurrence-negative group)

The two 1- to 3-year-old infants determined as being chicken egg allergy-negative as a result of heated chicken egg OFC had OVM-specific IgE IC50 values (OVM sIgE IC50 (nM)) of 4.39 nM and 194.71 nM measured by the DCP method which were higher than the cutoff value of 1.13 nM regarding the presence or absence of allergy in the IC50 value of OVM-specific IgE in 1- to 3-year-old subjects and were therefore confirmed to fall within the range of allergy negativity. Their values obtained by multiplying the OVM-specific IgE titer measured by the DCP method by the value of 1/IC50 were 757.12 (BUe/mL)/(nM) and 11.82 (BUe/mL)/(nM) which were lower than the cutoff value of 1556.70 regarding the presence or absence of allergy in OVM sIgE/OVMs IgE IC50 in 1- to 3-year-old subjects and were therefore confirmed to fall within the range of allergy negativity. In actuality, no sign of chicken egg allergy was confirmed in these two infants.

These two subjects also exhibited values higher than the cutoff value of 2.15 nM regarding the presence or absence of occurrence of anaphylaxis in the IC50 value of OVM-specific IgE (OVM sIgE IC50 (nM)) in 1- to 3-year-old subjects. Their values were also lower than the cutoff value of 1225.80 (BUe/mL)/(nM) regarding the presence or absence of occurrence of anaphylaxis using the parameter OVM sIgE/OVMs IgE IC50 for 1- to 3-year-old subjects, suggesting that the possibility of occurrence of anaphylaxis was also very low. At present, there is no report on the occurrence of anaphylaxis in these two subjects by the ingestion of chicken eggs.

Thus, the cutoff value regarding the presence or absence of occurrence of anaphylaxis determined by ROC analysis with the IC50 value of OVM-specific IgE as a parameter, and the cutoff value regarding the presence or absence of occurrence of anaphylaxis determined by ROC analysis with the value obtained by multiplying the OVM-specific IgE titer by the value of 1/IC50 as a parameter were confirmed to be effectively applicable to 1- to 3-year-old subjects.

Six 8- to 15-year-old children excluding THG-23 and THG-44 had OVM-specific IgE IC50 values (OVM sIgE IC50 (nM)) within the range of 1.73 nM to 16.03 nM measured by the DCP method which were higher than the cutoff value of 1.30 nM regarding the presence or absence of allergy in the IC50 value of OVM-specific IgE in 1- to 6-year-old subjects and were therefore confirmed to fall within the range of allergy negativity. Their values obtained by multiplying the OVM-specific IgE titer measured by the DCP method by the value of 1/IC50 were 170.93 (BUe/mL)/(nM) to 1082.41 (BUe/mL)/(nM) which were less than the cutoff value of 4459.00 (BUe/mL)/(nM) regarding the presence or absence of allergy in OVM sIgE/OVMs IgE IC50 in 1- to 6-year-old subjects and were therefore confirmed to fall within the range of allergy negativity.

Seven 8- to 15-year-old children excluding THG-44 all exhibited values higher than the cutoff value of 1.03 nM regarding the presence or absence of occurrence of anaphylaxis using the IC50 value of OVM-specific IgE in 1-to 6-year-old subjects as a parameter, and exhibited values lower than the cutoff value of 8960.00 (BUe/mL)/(nM) regarding the presence or absence of occurrence of anaphylaxis using the parameter OVM sIgE/OVMs IgE IC50 for 1- to 6-year-old subjects. Six of these 8- to 15-year-old children manifested no sign of occurrence of anaphylaxis. It was thus confirmed that the occurrence of anaphylaxis can be predicted using the cutoff value for 1- to 6-year-old subjects.

One 8- to 15-year-old child (THG-44) had an OVM-specific IgE IC50 value (OVM sIgE IC50 (nM)) of 0.93 nM measured by the DCP method which was less than the cutoff value of 1.30 nM regarding the presence or absence of allergy in the IC50 value of OVM-specific IgE in 1- to 6-year-old subjects and therefore fell within the range of allergy positivity. Also, the possibility of occurrence of anaphylaxis was predicted because the value thereof was less than the cutoff value of 1.03 nM regarding the presence or absence of anaphylaxis in the IC50 value of OVM-specific IgE in 1- to 6-year-old subjects.

In the THG-44 case, the value obtained by multiplying the OVM-specific IgE titer measured by the DCP method by the value of 1/IC50 was 15133.54 (BUe/mL)/(nM) which was higher than the cutoff value of 1556.70 regarding the presence or absence of allergy in OVM sIgE/OVMs IgE IC50 in 1- to 6-year-old subjects and thus fell within the range of allergy positivity. Also, very high possibility of occurrence of anaphylaxis was predicted because the value thereof was higher than the cutoff value of 8960.10 (BUe/mL)/(nM) regarding the presence or absence of anaphylaxis in OVM sIgE/OVMs IgE IC50 in 1- to 6-year-old subjects. Thus, this child is regarded as having the possibility of future occurrence of anaphylaxis, though no allergic symptom has not yet been confirmed by the ingestion of chicken eggs, and is under follow-up by dietary instruction, particularly, for chicken eggs under the supervision of a doctor.

One 8- to 15-year-old child (THG-23) had an OVM-specific IgE IC50 value (OVM sIgE IC50 (nM)) of 1.25 nM measured by the DCP method which was less than the cutoff value of 1.30 nM regarding the presence or absence of allergy in the IC50 value of OVM-specific IgE in 1- to 6-year-old subjects and therefore fell within the range of allergy positivity. However, low possibility of occurrence of anaphylaxis was predicted because the value thereof was higher than the cutoff value of 1.03 nM regarding the presence or absence of anaphylaxis in the IC50 value of OVM-specific IgE in 1- to 6-year-old subjects.

In the THG-23 case, the value obtained by multiplying the OVM-specific IgE titer measured by the DCP method by the value of 1/IC50 exhibited 942.36 (BUe/mL)/(nM) which was lower than the cutoff value of 4459.00 regarding the presence or absence of allergy in OVM sIgE/OVMs IgE IC50 in 1- to 6-year-old subjects and thus fell within the range of allergy negativity. Also, the value was lower than the cutoff value of 8960.10 (BUe/mL)/(nM) regarding the presence or absence of anaphylaxis in OVM sIgE/OVMs IgE IC50 in 1-to 6-year-old subjects. In the case of applying two cutoff values for 1- to 6-year-old subjects, this child is regarded as having low possibility of occurrence of anaphylaxis. This child is currently under follow-up because no allergic symptom appears by the ingestion of chicken eggs.

### (Conclusion about chicken egg allergy-negative group)

In the chicken egg allergy-negative (group (A)), it was confirmed that the cutoff value regarding the presence or absence of occurrence of anaphylaxis in the IC50 value of OVM-specific IgE, and the cutoff value regarding the presence or absence of occurrence of anaphylaxis in the value obtained by multiplying the OVM-specific IgE titer by the value of 1/IC50 can be effectively used. It was also confirmed that a cutoff value calculated on the basis of data on 1- to 6-year-old subjects can also be applied to 8-to 15-year-old children.

### (Chicken egg allergy-positive/anaphylaxis occurrence-negative group)

Two 8- to 15-year-old children (THG-31 and -47) determined as being chicken egg allergy-positive as a result of heated chicken egg OFC had OVM-specific IgE IC50 values (OVM sIgE IC50 (nM)) of 1.05 nM and 0.48 nM measured by the DCP method which were lower than the cutoff value of 1.30 nM regarding the presence or absence of allergy in the IC50 value of OVM-specific IgE in 1- to 6-year-old subjects and were therefore confirmed to fall within the range of allergy positivity.

Their values obtained by multiplying the OVM-specific IgE titer measured by the DCP method by the value of 1/IC50 were 1465.64 (BUe/mL)/(nM) and 3897.79 (BUe/mL)/(nM) which were lower than the cutoff value of 4459.00 (BUe/mL)/(nM) regarding the presence or absence of allergy in OVM sIgE/OVMs IgE IC50 in 1- to 6-year-old subjects and thus fell within the range of allergy negativity. These two subjects are likely to be in transition from chicken egg allergy positivity to negativity, albeit having mild symptoms of chicken egg allergy, and are under follow-up.

These children were predicted to have low possibility of occurrence of anaphylaxis because their values were much lower than the cutoff value of 8960.10 (BUe/mL)/(nM) regarding the presence or absence of anaphylaxis in OVM sIgE/OVMs IgE IC50 in 1- to 6-year-old subjects. In actuality, the children manifested no symptom of anaphylaxis. However, the THG-47 case had an IgE IC50 value lower than the cutoff value regarding the presence or absence of anaphylaxis and thus needs to be under future follow-up.

One 8- to 15-year-old child (THG-21) had an OVM-specific IgE IC50 value (OVM sIgE IC50 (nM) of 1.74 nM measured by the DCP method which was higher than the cutoff value of 1.30 nM regarding the presence or absence of allergy in the IC50 value of OVM-specific IgE in 1- to 6-year-old subjects and therefore fell within the range of allergy negativity. The value thereof obtained by multiplying the OVM-specific IgE titer measured by the DCP method by the value of 1/IC50 was 4897.47 (BUe/mL)/(nM) which was higher than the cutoff value of 4459.00 (BUe/mL)/(nM) regarding the presence or absence of allergy in OVM sIgE/OVMs IgE IC50 in 1- to 6-year-old subjects and therefore fell within the range of allergy positivity.

In the THG-21 case, the IC50 value was higher than the cutoff value of 1.03 nM regarding the presence or absence of anaphylaxis in the IC50 value of OVM-specific IgE in 1-to 6-year-old subjects, and the value obtained by multiplying the OVM-specific IgE titer by the value of 1/IC50 was lower than the cutoff value of 8960.10 (BUe/mL)/(nM) regarding the presence or absence of anaphylaxis in OVM sIgE/OVMs IgE IC50 in 1- to 6-year-old subjects. In the case of applying two cutoff values for 1-to 6-year-old subjects, this child is regarded as having low possibility of occurrence of anaphylaxis. In actuality, this child has light symptoms and has no history of developed anaphylaxis. Thus, it was confirmed that a cutoff value calculated on the basis of data on 1- to 6-year-old subjects can be applied to 8- to 15-year-old children.

One 8- to 15-year-old child (THG-13) had an OVM-specific IgE IC50 value (OVM sIgE IC50 (nM) of 2.7 nM measured by the DCP method which was higher than the cutoff value of 1.30 nM regarding the presence or absence of allergy in the IC50 value of OVM-specific IgE in 1- to 6-year-old subjects and therefore fell within the range of allergy negativity. The value thereof obtained by multiplying the OVM-specific IgE titer measured by the DCP method by the value of 1/IC50 was 317.17 (BUe/mL) / (nM) which was much lower than the cutoff value of 4459.00 (BUe/mL)/(nM) regarding the presence or absence of allergy in OVM sIgE/OVMs IgE IC50 in 1- to 6-year-old subjects and thus fell within the range of allergy negativity.

The THG-13 case had low possibility of occurrence of anaphylaxis because the value thereof was higher than the cutoff value of 1.03 nM regarding the presence or absence of anaphylaxis in the IC50 value of OVM-specific IgE in 1-to 6-year-old subjects. Also, low possibility of occurrence of anaphylaxis can be predicted because the value thereof was much lower than the cutoff value of 8960.10 (BUe/mL)/(nM) regarding the presence or absence of anaphylaxis in OVM sIgE/OVMs IgE IC50 in 1- to 6-year-old subjects. This child had no history of developed anaphylaxis, albeit having allergic symptoms, and is currently under follow-up because the OVM antibody titer itself is very low and allergic symptoms are observed to turn for the better.

### (Group with chicken egg anaphylaxis)

Study was conducted on a 10-year-old child who developed anaphylaxis. The IC50 value of OVM-specific IgE (OVM sIgE IC50 (nM)) measured by the DCP method was 0.26 nM which was lower than the cutoff value of 1.03 nM regarding the presence or absence of anaphylaxis in the IC50 value of OVM-specific IgE in 1- to 6-year-old subjects and was thus consistent with the situation of developed anaphylaxis. The value thereof obtained by multiplying the OVM-specific IgE titer measured by the DCP method by the value of 1/IC50 was 81174.34 (BUe/mL)/(nM) which was a numerical value much higher than the cutoff value of 8960.10 (BUe/mL)/(nM) regarding the presence or absence of anaphylaxis in OVM sIgE/OVMs IgE IC50 in 1- to 6-year-old subjects and was thus consistent with the situation of developed anaphylaxis.

Thus, in this 10-year-old child, it was also able to be confirmed that the cutoff value regarding the presence or absence of anaphylaxis in the IC50 value of OVM-specific IgE in 1- to 6-year-old subjects, and the cutoff value regarding the presence or absence of anaphylaxis in OVM sIgE/OVMs IgE IC50 in 1- to 6-year-old subjects can be applied to a criterion.

### (Conclusion)

It was confirmed that a risk of occurrence of anaphylaxis in an infant or a child can be predicted using a cutoff value determined and calculated by ROC analysis based on an antibody titer of an IgE antibody and avidity of the IgE antibody against an allergen. It was also confirmed that any one or a combination of a cutoff value regarding the presence or absence of anaphylaxis using the parameter OVM sIgE/OVMs IgE IC50, and a cutoff value regarding the presence or absence of anaphylaxis using IgE IC50 as a parameter, calculated on the basis of data on 1-to 6-year-old infants is applicable to 8- to 15-year-old children. The present invention is advantageous because an infant or a child who has not yet developed anaphylaxis, when found to have a value exceeding a cutoff value regarding the presence or absence of occurrence of anaphylaxis in, for example, [OVM sIgE/OVMs IgE IC50[(BUe/mL)/(nM)]] obtained by multiplying the OVM-specific IgE titer by the value of 1/IC50, or IgE IC50 (nM), can receive instruction for diet, etc. by a doctor or the like.

## Claims

1. A method for collecting data for predicting a risk of occurrence of anaphylaxis in an infant or a child having food allergy by using, as a criterion, a cutoff value determined by ROC analysis based on avidity of an IgE antibody against an allergen in a sample collected from the infant or the child.

2. The method for collecting data according to claim 1, wherein the cutoff value determined by ROC analysis based on avidity of an IgE antibody against an allergen is a cutoff value determined by ROC analysis based on a numerical value obtained by multiplying the avidity of an IgE antibody against an allergen by an antibody titer of the IgE antibody against the allergen.

3. The method for collecting data according to claim 2, wherein the avidity of an IgE antibody against an allergen is numerically converted as 1/IC50.

4. The method for collecting data according to claim 3, wherein the IC50 is a competitive binding inhibitory activity value.

5. The method for collecting data according to claim 1, wherein the cutoff value determined by ROC analysis based on avidity of an IgE antibody against an allergen is a cutoff value determined by ROC analysis based on IC50 of the IgE antibody.

6. The method for collecting data according to claim 5, wherein the IC50 is a competitive binding inhibitory activity value.

7. The method for collecting data according to any one of claims 2 to 6, wherein the antibody titer of the IgE antibody against the allergen is measured using a DCP chip.

8. The method for collecting data according to any one of claims 1 to 7, wherein the sample is plasma or serum.
